# EUROPEAN PATENT SPECIFICATION

(11) **EP 3 795 672 B1**
(45) Date of publication and mention of the grant of the patent: **20.11.2024**
(21) Application number: 19198072.1
(22) Date of filing: 18.09.2019
(51) Int. Cl.: C12M 1/34, A01N 1/02, C12M 1/00

(54) **SYSTEM AND METHOD FOR THE GENERATION OF HIGH CELL DENSITY SEED CULTURE**
SYSTEM UND VERFAHREN ZUR ERZEUGUNG EINER SAATKULTUR MIT HOHER ZELLDICHTE
SYSTÈME ET PROCÉDÉ DE GÉNÉRATION DE CULTURE DE GRAINES À HAUTE DENSITÉ CELLULAIRE

(43) Date of publication of application: 24.03.2021
(73) Proprietor: Sartorius Stedim Biotech GmbH, 37079 Göttingen (DE)
(72) Inventor: MATUSZCZYK, Jens-Christoph, 37079 Göttingen (DE); POLLARD, David James, South Boston, MA 02127 (US); LEMKE, Johannes, 37081 Göttingen (DE); RUHL, Sebastian, 63619 Bad Orb (DE)
(74) Representative: Vigand, Philippe

(56) References cited:
- WO-A1-2004/040976
- US-A1- 2017 112 122
- US-A1- 2019 223 433
- US-A1- 2019 274 299

## Description

The invention relates to a method and to systems for an automated production of High Cell Density Seed Culture (HCDC) preparations (HCDC-preparations) for cryo-preservation.

Often, production bioprocesses require so-called frozen seed cultures that are used to inoculate the seed culture of the bioprocess. HCDCs meet the need for shortening the seed train, i.e. making it more cost efficient. Hence, cryo-preserved HCDC-preparations, particularly HCDC-aliquots help improving production bioprocesses. HCDC-preparations can vary in quality. An important measure for the quality is the physiological status at which the HCDC experiences a cryo-treatment.

WO 2004/040976 A1 describes viral delivery systems and methods for infecting cells with viruses. The viral delivery systems may be cryogenically preserved and they may be used in the manufacture of vaccines, polypeptides, and polynucleotides. The invention includes a cryogenically protected viral delivery system for infecting host cells that comprises a cryogenic vessel and a plurality of virally infected cells in admixture with a cryo-protective agent contained in the cryogenic vessel.

US 2019/223433 A1 describes a method for the creation of a high-density cryopreserved cell bank using perfusion culture techniques and non-centrifugal concentration of cells. Methods of production using this high-density cryopreserved cell bank are also provided.

US 2017/112122 A1 describes methods of freezing mammalian cells for storage or improving thaw recovery of cell banks comprising freezing mammalian cells in a freezing medium having a pH of 6.7 to 8.5.

US 2019/274299 A1 describes the application of a viable cell monitor to the cryopreservation of plant cells. The viable cell monitor is, for the first time, used in a method for rapidly evaluating the effect of cell freezing-preservation. The effect of a cryoprotectant on cell preservation is evaluated through the change in the capacitance of viable cells before and after cell freezing and resuscitation.

According to an aspect of the disclosure, the object relates to improving the quality of HCDC-preparations before cryo-preservation.

The object is solved by systems and a method according to the independent claims. Preferred embodiments are covered by the dependent claims.

Before, the steps of production are completed, i.e. the steps of preparation of HCDC-preparations are completed, it is referred to a HCDC comprising HCDC-cells rather than a HCDC preparation.

According to the invention, a system is for an automated production of high cell density seed culture, HCDC, preparations, for cryo-preservation comprising
- a cell expansion device for growing a HCDC in a medium inside a device volume of the cell expansion device,
- at least one sensor for collecting data by at least temporally monitoring at least one parameter being indicative of the physiological state of the HCDC; and
- a control unit for processing the collected data to determine whether at least one predetermined criterion is fulfilled by the monitored parameter to control, based on whether the at least one predetermined criterion is fulfilled by the monitored parameter, at least a partial exchange of the medium by a cryo-protective agent, CPA.

The system allows for automatic replacement of the medium at least partially by the CPA. The system can provide HCDC-preparations for cryo-preservation of high quality. Preferably, all or at least most elements of the system can be controlled and/or regulated by the control unit, such that their functions can automatically be requested and/or initiated.

Monitoring a process online may help finding the ideal process time to initiate a treatment of the cells which are dispersed in a broth containing the HCDC, particularly a preparation of the HCDC for cryo-conservation. Further, the system can reproducibly start the preparation of the HCDC, i.e. the treatment of the HCDC cells, under predefined conditions, thus keeping alterations low between generations of different cell banks and/or cell stems or strains.

Continuous online, particularly *in-situ* monitoring allows for real-time process control. With off-line data, decisions may only be made at the time of sample analysis, resulting in imprecise timing or missing events entirely. Such on-line data may be collected by means of capacitance probes (also called capacitance sensors) and/or optical spectroscopy, pH-sensors, gas detectors etc. or a combination of several of the named sensors and probes.

Methods which are considered particularly useful, comprise optical probes that do not discriminate between viable and dead cells, capacitance probes and radio-frequency impedance (RFI) probes, wherein a capacitance probe and an RFI probe can detect living cells in a capacitance measurement.

Also, permittivity measurements may be useful because of the robust real-time data indicating the state of a viable cell density of HCDC. Permittivity measurements correlate well to off-line cell counting in the exponential phase without the risk of counting errors. The corresponding permittivity sensors can be used to increase process quality across many cell lines, process types, and scales.

Due to improved production of HCDC-preparations for cryo-preservation, namely stopping the growth at a point in time when the density of viable cells is particularly high, the system may help to reduce the cell expansion phase, i.e. the time period which is needed for a cell expansion. Hence, less medium is required and operational efforts and costs may be reduced as a consequence.

Further, the system helps keeping the cells in a beneficial condition by monitoring the physiological cellular state, for example the metabolism and the cell growth, and therefore the system may improve the performance and reproducibility of subsequent cultures. As a consequence, the production of a HCDC may be more reliable, provide a higher reproducibility, a better efficiency. In other words, the system has the advantage of reducing or even avoiding failure rates and of being more cost-efficient in the seed culture handling. The risk of a (high) variability and of performance fluctuations may hence be reduced or eliminated.

A further advantage of the system is given by the fact, that the number of steps, which need to be performed manually, can be reduced or even eliminated. The system may run the production of HCDC-preparations for cryo-preservation autonomously, for example overnight and/or over a long period and/or permanently without a person to interact with while providing an improved quality of HCDC-preparations with a high density of viable cells. A manual monitoring of the process, particularly a manual sampling of the HCDC and a determination of the viable cell density of the HCDC, can be avoided. Hence, the risk of contamination through a manual process step can be avoided or at least reduced.

These effects and advantages allow for a high (cost-) efficiency of the system and an easy and convenient handling. The handling may for example comprise merely providing the material required to start the production of HCDC-preparations including the HCDC growth and instructing the system to start the production. Further steps which may be performed by an operator may comprise presetting at least one predetermined criterion and potentially programming the system for specified functions.

Preferably, the at least one sensor comprises a capacitance probe, also called capacitance sensor, for collecting data by at least temporally monitoring at least one parameter comprising a capacitance, preferably online, i.e. substantially in real time during the measurement, wherein the parameter, particularly the capacitance is indicative of the physiological status of the HCDC.

The at least one parameter preferably comprises at least one of: the viable cell density or any parameter which is substantially indicative of the viable cell density, the overall cell density comprising viable and non-viable cells, a gas concentration, a gas pressure (for example oxygen and/or carbon dioxide), a capacitance, a permittivity, a resistance, a molecular vibrational absorption of light, an amount of scattered light, the pH, the amount and/or weight of bio mass, particularly of the HCDC.

The step of determining whether at least one predetermined criterion is fulfilled by the monitored parameter preferably comprises at least one of the following: determining whether the viable cell density of the HCDC exceeds or equals or falls below a threshold value, determining whether the viable cell density of the HCDC reaches a plateau value, determining whether the change of viable cell density of the HCDC in time exceeds, equals or falls below a predetermined value, determining whether the gas pressure inside the device volume reaches or falls below a predetermined value, determining whether the weight of HCDC exceed or equals or falls below a threshold value.

According to an aspect, the cell expansion device is arranged on a rocker which is configured to produce a movement of the cell expansion device, such that a liquid can be moved inside the device volume of the cell expansion device. According to an aspect, the cell expansion device is arranged on a magnetic stirrer which is configured to move a magnetic element inside the device volume such that a liquid can be moved inside the device volume. According to an aspect, the cell expansion device is arranged on a tempering device such as a heating plate configured to control, i.e. heat and/or cool a substance, for example a gas and/or a liquid inside the device volume. These aspects may be combined in one device. According to an aspect, the cell expansion device is arranged on a balance element which is configured to determine the weight of the cell expansion device.

It may be advantageous for the growth of the cells inside the device volume when the liquid, substantially the medium, in which they grow, is stirred or shaken to provide the for example with enough oxygen and to avoid an oxygen and/or nutrition depletion in their close vicinity. It may in addition be advantageous to provide a temperature control for tempering the medium with the cells to an optimal temperature for the growth. According to an aspect, the rocker, the magnetic stirrer and/or the tempering device are controlled by the control unit, such that the control unit can set parameters such as rocking strength, temperature and/or stirring frequency. Preferably, the control unit is configured to control the the rocker, the magnetic stirrer and/or the tempering device based on at least one criterion which is fulfilled or met by the monitored parameter. For example, the parameter may show a substantially exponential growth in a certain time window and a flattening in another time window. The control unit preferably is configured to determine when a certain degree of flattening is reaches by the parameter. As a response to the determined flattening, the control unit may decrease or increase the rocking frequency or rocking strength, or the control unit may decrease the temperature to substantially stop further cell growth.

According to another aspect, one of the at least one sensor is substantially arranged on or at the outside wall or skin of the cell expansion device. Alternatively or in addition, a sensor is substantially arranged on or at the inside wall or skin of the cell expansion device. For example, a gas sensor may be arranged on the inside wall of the cell expansion device. Alternatively or in addition a sensor is reaching from the outside into the inside device volume through the wall or skin of the cell expansion device.

According to the invention, corresponding to the unit required to implement the option A) of the method, the cell expansion device comprises
- at least one a drain for draining at least a portion of the medium or any other solvent from inside of the device volume towards the outside of the device volume; and
- a membrane element used for at least partial exchange of the medium by the CPA, wherein the membrane element is arranged at the drain, i.e. on and/or in and/or behind and/or before the drain, i.e. a drain hole or a port used as an outlet, such that the HCDC is retained by the membrane element inside the device volume when at least a portion of the medium is drained from the device volume through the membrane element and through the drain towards the outside of the device volume; and
wherein the control unit is configured to initiate, based on whether or not the criterion is fulfilled, for example when a threshold is reached by the viable cell density, the steps of
- draining at least a portion of the medium from inside the device volume through the membrane element to the outside of the device volume; and,
- adding the CPA to the HCDC inside the device volume, and
wherein the system comprises a transfer unit, controlled by the control unit, for transferring at least a portion of the HCDC with CPA from the device volume into one or more receiving devices to obtain HCDC-preparations for cryo-preservation.

The draining of at least a portion of the medium from inside the device volume through the membrane element to the outside of the device volume is preferably performed before the adding the CPA to the HCDC inside the device volume. Alternatively, it may however also be possible to perform both steps at least partially at the same time and/or to add the CPA after the draining of the medium. The latter is however less likely, as the CPA might also be drained from the device volume, which may be not wanted.

The system allows for automatic replacement of the medium at least partially by the CPA, in that the medium is drained through the membrane element and through the drain from the inside of the device volume to the outside, wherein the HCDC is retained inside the device volume while a CPA is added to the HCDC substantially inside the device volume, simultaneously or afterwards.

A membrane element may be used not only for the at least partial exchange of the medium by the CPA but also for draining waste substances from the inside of the device volume towards the outside, or for rinsing purposes.

These steps may for example be done substantially at the same time, such that the concentration of viable cells inside the device volume remains substantially stable over the time of exchanging at least a portion of the medium by the CPA. This might be advantageous, as cells might potentially denature if the concentration is becoming too high.

The system can provide HCDC-preparations for cryo-preservation of high quality. Preferably, all or at least most elements of the system can be controlled and/or regulated by the control unit, such that their functions can automatically be requested and/or initiated.

Further, the system helps keeping the cells in a beneficial condition by monitoring the physiological cellular state, for example the metabolism and the cell growth, and therefore the system may improve the performance and reproducibility of subsequent cultures. As a consequence, the production of a HCDC may be more reliable, provide a higher reproducibility, a better efficiency. In other words, the system has the advantage of reducing or even avoiding failure rates and of being more cost-efficient in the seed culture handling. The risk of a (high) variability and of performance fluctuations may hence be reduced or eliminated.

The transfer unit is configured to automatically transfer at least a portion of the HCDC with CPA from the device volume into one or more receiving devices to obtain HCDC-preparations for cryo-preservation. In other words, transfer into receiving devices can be conducted using an automated process.

Automatically transferring the HDCD with CPA into a device volume particularly refers to autonomously transferring, i.e. the transfer unit is not required to be called or instructed to perform the step of transferring but performs this step independently. Alternatively, the transfer step may be initiated by the control unit. Alternatively or in addition, at least one switch controls the flow of substance from the cell expansion device towards at least one portion of the transfer unit. The switch may be controlled manually and/or automatically and/or by a command of the control unit.

According to an aspect, multiple elements of the system exchange data with the control unit and/or are controlled by the control unit. Particularly, at least two out of the following exchange data with the control unit and/or are controlled by the control unit: the cell expansion device, one of the units having a rocker function, a balance function, a heating function and/or a cooling function, the transfer unit, at least one sensor, at least one flow control, at least one switch, at least one port and/or at least one valve.

According to the invention, corresponding to the unit to implement option B) of the method, the system further comprises
- a centrifuge unit for centrifuging the HCDC with the medium, wherein centrifuge unit is used for at least partial exchange of the medium by the CPA;
- at least one transfer unit, controlled by the control unit, for transferring at least a portion of the HCDC with the medium into at least one centrifuge container of the centrifuge unit; and
- wherein the control unit is configured to initiate, based on whether or not the criterion is fulfilled, the steps of transferring at least a portion of the HCDC with the medium from the device volume into the at least one centrifuge container of the centrifuge unit, centrifuging with the centrifuge unit the transferred HCDC with the medium, separating at least partially the centrifuged medium from the HCDC, and adding the CPA to the HCDC.

In other words, a system is for an automated production of HCDC-preparations for cryo-preservation, comprising
- a cell expansion device, preferably a bag, such as a single-use bag, for growing a HCDC dispersed in a medium inside a device volume of the cell expansion device;
- at least one sensor, particularly a capacitance probe for collecting data by at least temporally monitoring at least one parameter, particularly a capacitance of the HCDC, wherein the parameter is indicative of a physiological status of the HCDC;
- a centrifuge unit, also referred to as centrifuge, for centrifuging the HCDC with the medium, preferably comprising at least one centrifuge container, preferably several centrifuge tubes;
- a first transfer unit, such as a tubing system, controlled by the control unit, for transferring at least a portion of the HCDC with medium into at least one, preferably several centrifuge containers of the centrifuge unit; and
- a control unit for processing the collected data to determine whether at least one predetermined criterion is fulfilled by the monitored parameter, wherein the control unit is configured to initiate, based on whether or not the criterion is fulfilled, the steps of transferring at least a portion of the HCDC with the medium via the first transfer unit from the device volume into the at least one centrifuge container of the centrifuge unit, centrifuging with the centrifuge unit the transferred HCDC with the medium, separating at least partially the centrifuged medium from the HCDC or the HCDC from the medium, and adding a cryo-protective agent, CPA, to the HCDC; and optionally
- a second transfer unit, controlled by the control unit, for transferring at least a portion of the HCDC with CPA from the centrifuge unit into one or more receiving devices to obtain HCDC-preparations for cryo-preservation.

The present aspect has the advantage that cells are separated from the medium by centrifuging the cells with the medium in the centrifuge. After a physical separation, for example by sucking out at least a portion of the medium with a needle which is preferably performed by the control unit, controlling a needle for sucking out the medium, the CPA is added to the cells. This aspect represents an alternative or supplementary aspect to the separation according to the first aspect, which is performed via the membrane element. Both aspects may be combined in one single system. In other words, the centrifuge is optional, if CPA is added in the bag, no centrifuge is required, however, it may still be existent in the system but not used. It is however also not excluded that a case may arise in which options A) and B) are applied to produce a HCDC preparation.

The system comprises at least one transfer unit which may be configured to transfer the HCDC with the medium into one or more centrifuge containers, such as centrifuge tubes, which are provided inside the centrifuge. The centrifuge tubes inside the centrifuge are used for centrifuging the HCDC with the medium. The medium may be removed at least partially, for example drained from the centrifuge containers and CPA is added into the same centrifuge container which is later also used for the cryo-treatment. If several centrifuge containers are also used for the cryo-treatment, the step of transferring the HCDC with medium into the centrifuge containers may equal a step of aliquoting substantially the produced and prepared volume of HCDC, as the produced volume of HCDC is partitioned into smaller portions.

In addition, the system may comprise a second transfer unit, controlled by the control unit, for transferring at least a portion of the HCDC with CPA from the centrifuge unit, particularly from at least one centrifuge containers into one or more receiving devices. The CPA may be added to the HCDC inside the centrifuge container and/or inside the receiving device to obtain HCDC-preparations for cryo-preservation. In this case, the centrifuge container is not identical with the receiving device. The receiving device may comprise at least one of a vial, a container and a tube.

According to the invention, the system comprises the units combining the options A) and B), a particular advantage may arise. It may for example be the case that some cell types may be better suited for one of the system types whereas other cell types might be well suited for the other system. For example, a concentration of the HCDC may be very well controlled if a substance is drained via the membrane element while another substance is refilled to replace the drained substance and to control the cell density. Some cells may be particularly sensitive and hence, the membrane technique might be preferred in such a case. For other types of cells, it may the system comprising the centrifuge unit may be better suited. To provide a high degree of versatility, it is hence advantageous to combine both aspects such that the system may be programmed to choose the better suited technique, i.e. the membrane or the centrifuge technique. According to an aspect, the control unit is configured, based on the collected data, to decide which technique is best suited for the HCDC. Alternatively, an operator may meet that decision.

According to an aspect, the sensor forms an integral unit with the cell expansion device), preferably, wherein the sensor is welded into at least a portion of a wall of the cell expansion device and/or wherein the sensor inserts into the device volume through a port of the cell expansion device.

Preferably, the sensor reaches through the wall of the cell expansion device via a port into the device volume, where the sensor can substantially physically contact the substance. Preferably, the sensor is a capacitance probe for detecting the capacitance of the broth with the HCDC. Preferably, the wall of the cell expansion device is at least partially the skin of a bag, particularly a single-use bag.

According to an aspect, the membrane element forms an integral unit with the cell expansion device.

It is an advantage to provide elements as integral units with the cell expansion device due to a specific purpose or function of the cell expansion device. The cell expansion device may be a single use cell expansion device, wherein the element which is formed integrally with the cell expansion device may also be a single use element, such that the entire cell expansion device together with the integral element may be disposed after use. The cell expansion device being formed integrally with at least one element may be configured for sterilization or disinfection. The risk of contamination may hence be reduced. According to a preferred aspect, a capacitance sensor is formed integrally with the cell expansion device, particularly arranged on the outside or inside skin or wall of the cell expansion device. According to a preferred aspect, the cell expansion device comprises a bag substantially comprising the device volume, particularly a single-use bag.

Alternatively, elements such as the sensor or the membrane may be removed from the cell expansion device and/or added to the cell expansion device. Hence, elements which are not configured for single-use, may be reused. Further, elements may be sterilized apart from the cell expansion device, possibly due to different material requirements.

According to another aspect, the sensor comprises at least one out of: a capacitance sensor, an optical sensor, a pH-sensor, a gas sensor, a permittivity sensor, a radio-frequency impedance (RFI) sensor or the like, wherein an optical sensor may be configured for Raman, Infrared and/or UV-vis spectroscopy. According to an aspect, the sensor used a substantially non-invasive method which does substantially not influence or change the physical, chemical and/or biological parameters inside the cell expansion device. Particularly, the sensor is configured to monitor at least one parameter without physically contacting a substance, particularly a gas and/or a liquid inside the cell expansion device. Particularly, the sensor is configured to monitor at least one parameter from the outside of the cell expansion device.

It may be desired to precisely monitor the physiological status of the HCDC, hence, it is advantageous to monitor several parameters which complementary indicate the physiological status of the HCDC. Hence, misinterpretations of one parameter may be avoided, as the second parameter may provide addition information to interpret the status.

The system has the advantage that the sensor is capable of adding benefit to the process chain as the process can be online monitored. This allows for versatility and freedom to operate in the field of HCDC frozen seed culture generation.

According to an aspect, the membrane element comprises at least one of: a membrane filter, a dialysis membrane, a hydrophilic filter, a hydrophobic filter, a microfilter, a nanofilter. The membrane element may be configured for filtration, microfiltration, ultrafiltration or nanofiltration, wherein filtration is considered to be performed with a pore size of approximately 10 µm or bigger, wherein microfiltration is considered to be performed with a pore size of approximately 0,1 µm or bigger, wherein ultrafiltration is considered to be performed with a pore size of approximately 100 nm to 2 nm and wherein nanofiltration is considered to be performed with a pore size of approximately 2 nm to 0,5 nm. A membrane element as described in here is preferably configured to retain all the cells, viable of not, inside the device volume, when a substance is drained outside from that device volume. Hence, as cells can have typical diameters of approximately 1 to 30 µm, the membrane element is preferably configured to allow at least a microfiltration or an ultrafiltration.

Preferably, the membrane element is substantially impermeable for the HCDC and substantially permeable for liquids, for the medium, for feed media and the CPA. Hence, the HCDC can be retained when a liquid is drained from the device volume.

According to a further aspect, the control unit is configured to, based on the monitored parameter, automatically control at least one second parameter, wherein the second parameter is preferably configured to control and/or regulate the physiological status of the HCDC.

Particularly, the at least one second parameter is a system specific parameter which is substantially not indicative of the physiological status of the HCDC but may regulate the physiological status. Preferably, the at least one second parameter comprises at least one of: a rocking frequency, a stirring frequency, a temperature, a concentration of feed medium, a salt concentration for example of the medium, an oxygen concentration, a proton concentration, a pressure, a light to which the HCDC can be exposed etc. For example, in view of the above, a second parameter may be controlled, for example by the control unit, in that the rocking and/or a stirring frequency, the temperature and/or the pressure is or are controlled. Particularly, at least one of the following may be controlled, preferably by the control unit: influx into the device volume of a fresh medium, a feed medium, a pH correctant, a gas, such as oxygen, a buffer, an aqueous salt solution or other substances which may be suitable to control the physiological state of the HCDC.

According to a further aspect, at least one out of the cell expansion device, the sensor, the centrifuge unit the receiving devices comprising a bag, a tube and/or a vial, and at least a portion of the transfer unit is or are one or more single-use element and/or a single use consumable. A part of the system may also be a single-element which may be for reuse, for single-use, for removal and/or exchange.

A single-use centrifuge or any other element which is constructed as a single-use element, reduces the risk of contaminating the substance inside the device volume. The single-use centrifuge and/or element is particularly configured to be sterilized.

According to the invention, a method is for an automated production of high cell density seed culture preparations, HCDC-preparations, for cryo-preservation, comprising the steps of
- growing a HCDC in a medium inside a device volumeof the cell expansion device;
- collecting data by at least temporally monitoring at least one parameter wherein the parameter is indicative of a physiological status of the HCDC;
- sending the collected data to a control unit;
- processing by the control unit the collected data at least comprising determining whether at least one predetermined criterion is fulfilled by the monitored parameter; and
- controlling, based on whether the at least one predetermined criterion is fulfilled by the monitored parameter, at least a partial exchange of the medium by a cryo-protective agent, CPA.

All previously mentioned technical effects and advantages may apply if the according features and aspects are reflected herein.

According to the invention, the method comprises, based on whether or not the monitored parameter fulfills the at least one criterion:
- initiating by the control unit the steps comprising
   Option A)
      - draining via a membrane element for retaining the HCDC inside the device volume and via a drain at least a portion of the medium from the device volume,
      - adding a cryo-protective agent, CPA, to the HCDC in the device volume, and
      - transferring at least a portion of the HCDC in the CPA from the device volume into one or more receiving devices to obtain HCDC-preparations for cryo-preservation; and/or
   Option B)
      - transferring at least a portion of the HCDC with the medium from the device volume into at least one centrifuge container of the the centrifuge unit,
      - centrifuging with the centrifuge unit the transferred HCDC with the medium,
      - separating at least partially the centrifuged medium from the HCDC, and
      - adding CPA to the HCDC to obtain HCDC-preparations for cryo-preservation, preferably, wherein
   transferring at least a portion of the HCDC (2) with the CPA (a) from the centrifuge container into one or more receiving devices (7a) to obtain HCDC-preparations for cryo-preservation, wherein the step of transferring at least a portion of the HCDC (2) with the medium (b) from the device volume (V) into at least one centrifuge container and/or transferring at least a portion of the HCDC (2) with the CPA (a) from the centrifuge container into one or more receiving devices (7a) is performed automatically.

In other words, according to an aspect, a method is for an automated production of high cell density seed culture preparations, HCDC-preparations, for cryo-preservation, comprising the steps of
- growing a HCDC in a medium inside a device volume of the cell expansion device;
- collecting data by at least temporally monitoring at least one parameter wherein the parameter is indicative of a physiological status of the HCDC;
- sending the collected data to a control unit;
- processing by the control unit the collected data at least comprising determining whether at least one predetermined criterion is fulfilled by the monitored parameter;

Based on whether or not the monitored parameter fulfills the at least one criterion:
- initiating by the control unit the steps comprising
   Option A) comprising:
      o draining through a membrane element for retaining the HCDC inside the device volume and through a drain at least a portion of the medium from the device volume,
      o adding CPA to the HCDC, i.e. providing the HCDC with a cryo-protective agent, CPA, in the device volume, which may be filling CPA into the device volume to the HCDC, and
      o transferring at least a portion of the HCDC in the CPA from the device volume into one or more receiving devices to obtain HCDC-preparations for cryo-preservation; and/or
   Option B) comprising:
      o transferring at least a portion of the HCDC with the medium from the device volume into at least one centrifuge container of the the centrifuge unit,
      o centrifuging with the centrifuge unit the transferred HCDC with the medium,
      o separating at least partially the centrifuged the medium from the HCDC, and
      o adding CPA to the HCDC, i.e. providing the HCDC with the CPA, which may be filling CPA into the centrifuge container to the HCDC and/or filling CPA into a receiving container if the HCDC is transferred into a receiving container, to obtain HCDC-preparations for cryo-preservation.

Referring to option B), the step of adding the CPA to the HCDC may comprise filling the CPA into the centrifuge container to the HCDC, particularly when the centrifuge container is used for storage and/or cryo-treatment. Alternatively or in addition, the step of separating at least partially the centrifuged the medium from the HCDC may comprise transferring the HCDC at least partially from the at least one centrifuge container into at least one receiving device, optionally comprising aliquoting at least apportion of the HCDC. The CPA may be added, in this particular case, to the HCDC for example, when the HCDC is inside the at least one centrifuge container and/or inside the receiving device.

According to a further aspect, the method further comprises, based on the monitored parameter, automatically controlling at least one second parameter, wherein the second parameter is preferably configured to control and/or regulate the physiological status of the HCDC.

According to a further aspect, the method further comprises based on the monitored parameter, automated controlling, by the control unit, of at least one second parameter, wherein the second parameter is configured for controlling and/or regulating a physiological status of the HCDC.

According to a further aspect, the processing of the collected data comprises automatically analyzing the collected data by fitting a function and/or model building afunction to at least a portion of the collected data to obtain a fit result.

Automatically fitting a function according to a theory, helps easily analyzing the collected data. As an example, a theory may for example comprise an exponential growth of cells for a certain period of time. Hence, for example, at least a portion of the collected data which reflect the cell growth in time, can be analyzed by fitting an exponential function to them. Based on the fit result, a prediction and/or an assessment of the growth can be made, for example. Based on this, a function of an element of the system according to any one aspect may be called. For example, an influx of feed medium may be initiated by the control unit.

According to a further aspect, the determining whether at least one predetermined criterion is fulfilled by the monitored parameter is based on automatically analyzing of the fit result.

Analogously, one of the the systems according to previous aspects, particularly the control unit of such systems is configured to determine whether at least one predetermined criterion is fulfilled by the monitored parameter based automatically analyzing the collected data by fitting a function to at least a portion of the collected data to obtain a fit result and preferably, automatically analyzing the fit result.

For example, the step of analyzing the fit result may comprise calculating at least one derivative and comparing the derivative with a predetermined value. If the derivative exceeds the predetermined value, the control unit may cause an action such as initiating an influx of fresh medium and/or a feed medium and/or CPA into the device volume and/or terminate the cell growth by causing a cooling down of the device volume and the HCDC.

According to a further aspect, the method further comprises the step of cryo-preserving the HCDC-preparations, i.e. substantially freezing and/or cooling down the HCDC with the CPA in the one or more receiving devices to obtain cryo-preserved HCDC, such as cryo-preserved aliquots of HCDC in tubes.

Analogously, the system may comprise a cryo-preserving unit for cryo-preserving the HCDC-preparations.

The method or the systems according to any one of the above aspects may hence comprise a step, particularly an automated step of cryo-treatment. If this step is automated, it has the advantage of fulfilling precisely preset conditions, which for example were found to be optimal. The reproducibility to obtain high quality cryo-preserved HCDC is particularly high. For example, the quality of the cryo-preserved HCDC may depend on an exact cryo-treatment protocol, which may include the time which passes between producing or preparing the HCDC-preparations and the cryo-treatment, and/or a stepwise or immediate or instant cryo-treatment procedure.

According to a further aspect, the method further comprises further comprises, in at least one iteration step, determining the quality of the obtained HCDC-preparations and
- varying the predetermined criterion to be fulfilled by the monitored parameter and/or
- varying at least one second parameter; and
- repeating the method according to any aspect, particularly until an optimal quality is obtained.

The quality of the obtained HCDC-preparations may for example comprise a viable cell density of a cryo-preserved HCDC-preparation after defrosting. The predetermined criterion may for example be a threshold value which is exceeded by the viable cell density right before the cryo-treatment. The threshold value might not necessarily linearly correlate with the quality. Hence, this threshold value may be varied to determine at which threshold value the best quality after defrosting can be obtained.

According to another aspect, the method further comprises at least one iteration step of determining the quality of the obtained HCDC-preparations before or after cryo-preservation and varying at least one of the at least one second parameter(s) and repeating the method according to any aspect, particularly until an optimal quality is obtained.

It is an advantage to provide a system and a method which automatically determine ideal conditions under which high quality HCDC-preparations are produced. For example, an operator may request the system to vary the temperature, the amount or concentration of added CPH, the amount of added feed medium, the amount of added oxygen, the salt and/or the nutrition concentration and/or any other relevant parameter which can influence the physiological state and the quality of the HCDC-preparations before or after cryo-treatment. Hence, the method according to the present aspect and the related systems are high in precision and help finding the optimal conditions under which high quality HDC can be obtained.

The above aspects are particularly well suited for mammalian cell culture based bioprocesses using online sensors to determine the ideal time point for initiating the treatment of cells for cryo-preservation. Given a good correlation of cell density to online sensor signals (like capacitance) other possible areas of application can be considered, such as microbial applications and/or stem cell applications.

In the following, some example embodiments will be described in detail, wherein the invention should not be understood to be limited to the example embodiments described. Single features being described in a particular embodiment may be arbitrarily combined, given that they are not excluding each other. In addition, different features which are provided together in the example embodiments are not to be considered restrictive to the invention.

Brief description of the drawings, noting that figure 1 does not correspond to the claimed invention and figures 2-6 correspond to systems according to the invention or to units involved in the options A) and/or B) of the method:
**Fig. 1** is a schematic view of a system for an automated production of (cryo-preserved) HDCD-preparations not corresponding to the invention;
**Fig. 2** is a schematic view of a system for an automated production of (cryo-preserved) HCDC-preparations according to another embodiment;
**Fig. 3** is a schematic view of a system for an automated production of (cryo-preserved) HCDC-preparations according to a further embodiment;
**Fig. 4** is a schematic view of a part of a system for an automated production of (cryo-preserved) HCDC-preparations according to an embodiment;
**Fig. 5** is a schematic view of a further part of a system for an automated production of (cryo-preserved) HCDC-preparations according to an embodiment;
**Fig. 6** is a schematic view of a part of a system for an automated production of (cryo-preserved) HCDC-preparations according to another embodiment;
**Fig. 7** is a schematic view of the method for an automated production of (cryo-preserved) HCDC-preparations; and
**Fig. 8** is an exemplary plot of viable cell concentration depending plotted against the measured capacitance.

Detailed description of example embodiments:

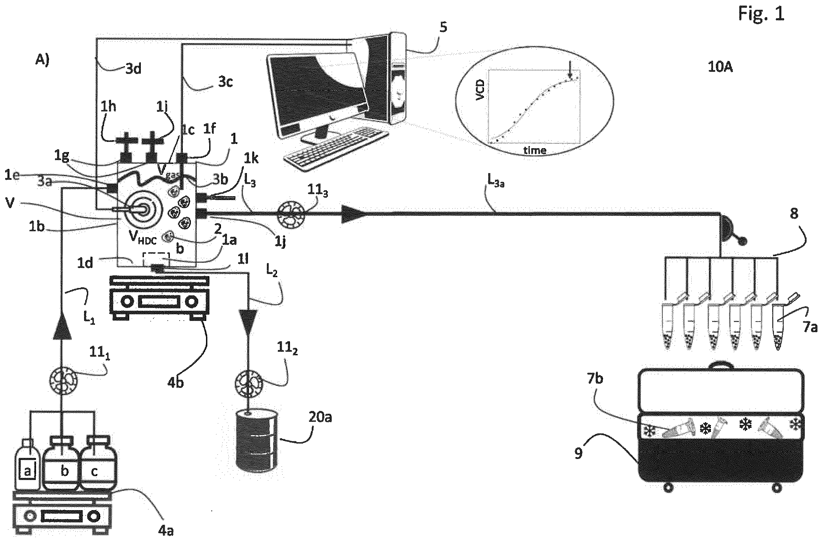

**Fig. 1** is a schematic view of a system 10A for an automated production of (cryo-preserved) HCDC-preparations which comprises the option A) in which a membrane element 1a is used to produce the HCDC-preparations. An extension device 1 is provided on a unit 4b, preferably a rocker having a rocking function, which may be combined with a heating and/or cooling function, a balance function, and/or a magnetic stirrer function. The unit 4b may be controlled by the control unit 5 and may send data to and/or receive data from the control unit 5.

The extension device 1 comprises ports 1e, 1k used as an inlet for example for liquid and/or gaseous substances, a port 1l used as an outlet for cell-free substances and ports 1j, 1k for used as outlets for cell-containing substances. The port 1j is connected fluidly with a path 11₃, for example a tubing. Ports 1h, 1i are used as inlets and/or outlets mainly for gaseous substances. Another port 1i is used for a second sensor 3b. Further ports may be arranged on the extension device 1, for example a port for an optical measurement may also be arranged on the extension device 1. All ports may be opened and/or closed manually and/or automatically, controlled by the control unit 5. All ports may comprise valves and/or filters, opening- and/or closing mechanisms.

The extension device 1 comprises a skin 1b or wall which comprises a bottom section 1d and a top ceiling section 1c. The extension device 1 comprises a device volume V for receiving and storing the HCDC 2 and wherein the HCDC 2 can grow to reach the preferred viable cell density. The HCDC cells 2, for simplicity merely called HCDC 2, may be dispersed in a medium b. The substance comprising the HCDC 2 and the medium b may be considered a so-called broth. The the broth has a volume V_{HCDC} which may equal the device volume V if the broth is substantially completely filling the device volume V. It may however be likely that also a gas is taking up space in the device volume V. The gas may stand above the broth and have a volume V_{gas}. The gas may enter or exit through an inlet and/or outlet 1g. The inlets and/or outlets may have at least one valve function to let a gas only through in one direction, either inwards or outwards. A gas pressure may be built up by filling a gas through an inlet 1g into the device volume V and the gas pressure may be reduced by releasing the pressure through an outlet 1g or by sucking gas out through the outlet 1g. The gas may comprise for example oxygen and/or CO₂. The inlets and/or outlets 1g may comprise filter elements 1i, 1h. The filter elements 1i, 1h may for example preserve the device volume V from contaminations from the outside.

The device volume V is fluidly connected by at least one port 1e to reservoirs of substances a, b, c: a fresh liquid culture medium b, denoted medium for simplicity, a base and/or a nutrition feed medium c and a CPA. The reservoirs of the substances a, b, c share a common port 1e connected by a common path L₁ into the device volume V. The reservoirs of the substances a, b, c may alternatively be fluidly connected with the device volume V by individual branches, which are connected to individual ports on the cell extension device 1. The substances a, b, c may flow into the device volume V individually and on demand, for example, when the control unit 5 initiates a flow of each individual substance a, b, c, at different times and/or at the same time. The reservoirs of the substances a, b, c are arranged on a unit 4a which may have at least one of the following functions: rocking, heating, cooling, balance and magnetic stirring. The unit 4a may be controlled by the control unit 5 and may send data to and/or receive data from the control unit 5. For example, the control unit 5 may cause the flow control unit 11₁ to specifically drive the CPA (substance a) from the reservoir into the device volume V as a consequence when the predetermined criterion is fulfilled by a parameter. The predetermined criterion may for example be a preset or predetermined threshold of a viable cell concentration. When the parameter, which may be a capacitance, indicates that the viable cell concentration surpasses the threshold, the flow of CPA (substance a) into the device volume V is initiated by the control unit. Before this step, the control unit 5 may heat the CPA (substance a) to a suitable temperature by means of the unit 4a having a heating function in that particular case, for example.

The physiological state of the HCDC 2 may be monitored at least over a period of time by means of the electrode sensor 3a which is provided on or at the skin 1b of the cell expansion device 1. The electrode sensor is a capacitance sensor 3a and is configured to record a capacitance which is indicative of the physiological state of the HCDC 2, since the capacitance substantially linearly correlates in at least a capacitance range with the viable cell density. The capacitance sensor 3a comprises a data transfer unit 3d for transferring the collected data comprising the capacitances measured in time to the control unit 5. The data transfer unit 3d may be a data cable or a wireless connection, for example Bluetooth or internet or the like.

Further, at least a second sensor 3b is used to monitor at least one further parameter. In the present **Fig. 1****,** the second sensor 3b reaches through a sensor port 1f from the outside of the skin 1b to the inside of the device volume V. The second sensor 3b is arranged on the top ceiling section 1c. In the present case, the second sensor 3b may be a pH sensor which is configured to monitor a pH inside the broth. The second sensor may provide complementary information in addition to the capacitance sensor 3a. The information may help to assess the physiological status of the HCDC 2 more precisely and/or may help to predict changes due to known correlations between the second parameter, such as the pH and the physiological status of the HCDC 2. The second sensor 3b also comprises a data transfer unit 3c for transferring the collected data comprising the second parameter such as the pH measured in time to the control unit 5. The data transfer unit 3c may also be a data cable or a wireless connection, for example Bluetooth or internet or the like.

Particularly, the system 10A being based on option A) comprises a membrane element 1a, which may in the present example be a membrane filter arranged substantially inside the device volume V to substantially separate the HCDC from the drain 1l, which is a port for cell-free harvest. The drain is arranged on the bottom section of the extension device 1 to substantially fluidly connect the device volume with the outside. The membrane element 1a however retains the HCDC inside the device volume V when a substance of the broth, for example a medium b is drained from the inside of the device volume V outwards. The substance which is drained is guided by a path L₂ into a container 20a, for example a bin or a container for recycling the substance. The flow of the substance may be controlled by the second flow control 11₂. The second flow control 11₂ may be configured to produce an underpressure for sucking out the substance through the drain 1l and the membrane element 1a. The second flow control 11₂ may also be controlled by the control unit 5.

When the control unit 5 initiates a cell harvesting, for example when a threshold of a viable cell density is reached or surpassed, the port 1j may be opened and the substance, preferably containing HCDC 2 and CPA (substance a) can be guided along a path L₃ₐ from the device volume V to a distribution unit 8. A third flow control unit 11₃ may be configured to produce an underpressure for sucking out the substance through the port 1j. The third flow control 11₃ is preferably also controlled by the control unit 5. The distribution unit 8 aliquots, i.e. distributes the substance containing HCDC 2 and CPA (substance a) in equal volume partitions into tubes 7a which are used for storage and for freezing of substances. The control unit 5 may control the amount and the ratio in which the substance is partitioned. Further, a mechanism controlled by the control unit 5 may seal the tubes 7a to prevent that the substance to leak out of the tubes 7a. The tubes containing the substance which comprises HCDC and CPA may be considered HCDC-preparations, particularly HCDC-aliquots for cryo-preservation. The HCDC-preparations are then cryo-treated by freezing the single tubes with the substance using a cryo-unit 9, which is a freezer in the present case. The cryo-treated HCDC-aliquots may be considered cryo-preserved HCDC preparations 7b. The cryo-preserved HCDC preparations 7b may later be defrosted on demand to make use of the cells for other purposes.

The option A) as illustrated in **Fig. 1** is based on the steps of draining a cell-free substance, such as a medium b, from the device volume V via the membrane element 1a and the drain 1l to the outside, whereas a CPA (substance a), for example a CPA solution, such as DMSO is filled from the reservoir into the device volume V to the HCDC 2 to at least partially replace the volume of the substance which has been drained from the device volume V. The product of this procedure may substantially be a mix of HCDC and CPA or it may substantially be a mix of CPA (substance a), HCDC and the medium (substance b) and/or residuals of a base and/or a nutrition feed (substance c).

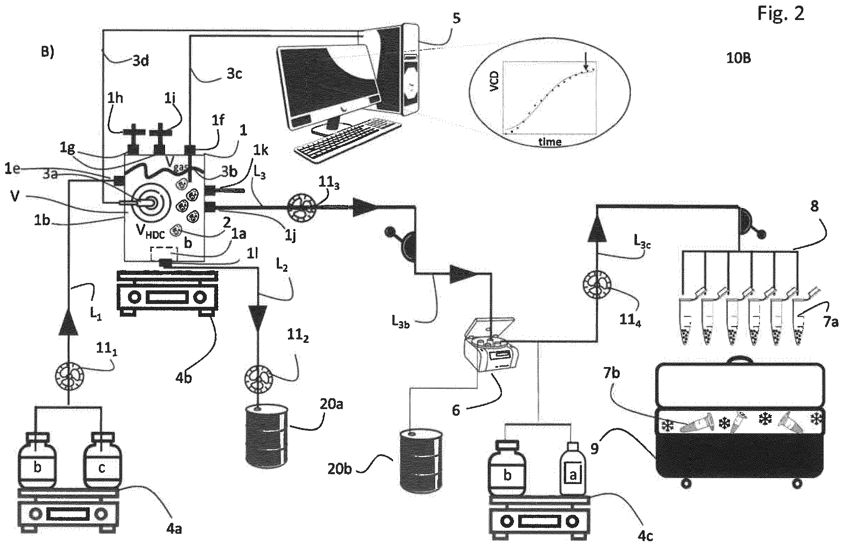

**Fig. 2** is a schematic view of a system 10B for an automated production of (cryo-preserved) HCDC-preparations according to another embodiment. The system 10B relies on the option B) which is based on the use of a centrifuge to separate at least a portion of the medium b from the HCDC 2 to which CPA (substance a) is added. The present exemplary system 10B however also comprises a membrane element 1a which is however in the pure option B) not used for separating the medium b from the HCDC 2. Hence, the present system 10B does not comprise a fluid connection between the device volume V and a reservoir of the CPA (substance a). Merely, the substances b and c, a medium and a base and/or a nutrition feed, i.e. a feed medium, can be transferred into the device volume V. If the control unit 5 is required to stop or to slow down the growth, the substance inside the device volume V comprising for example a feed medium c, may be drained cell-free from the device volume V via the drain 1l and the membrane element 1a. In an alternative, the cell extension device 1 may as well be a cell extension device 1 without a membrane element 1a.

In a moment of time, which is well-suited for harvesting the cells of the HCDC 2, for example when a high viable cell concentration is reached, the control unit 5 initiates a flow of the substance comprising HCDC 2 and the medium b from the device volume V via a path L₃ and L_{3b} into at least one or more centrifuge container of a centrifuge, wherein the centrifuge containers are preferably arranged inside the centrifuge. The control unit 5 may control the third flow control 11s to produce an underpressure to cause the substance to flow. The centrifuge containers may for example comprise centrifuge tubes.

The centrifuge 6 is controlled by the control unit 5. The control unit 5 particularly initiates the step of centrifuging the substance to separate the phases of the HCDC 2 and the medium b from each other. Preferably, the centrifuge 6 is a single-use centrifuge. The medium b is at least partially physically separated from the HCDC 2, i.e. guided from the centrifuge container into another container 20b outside the centrifuge 6. This is particularly, also controlled by the control unit 5. The CPA (substance a) is added to the HCDC 2 after removing at least a portion of the medium into the container 20b. Potentially, a fresh medium and/or a buffer solution is also added to the HCDC 2. This may be performed for example by addition into the centrifuge containers. In the present case, substances a, b which can be added to the centrifuged HCDC are arranged on a unit 4c which may be configured to condition the substances a, b. The unit 4a may particularly act as a rocker and/or a balance and/or a magnetic stirrer and/or temperature regulator.

After the addition of the CPA (substance a) the control unit 5 may cause a flow of the substance comprising HCDC 2 and CPA, regulated by the fourth flow control 11₄ to be guided to the distribution unit 8.

Alternatively, the vials, such as the centrifuge tubes are also used for the cryo-treatment of the HCDC-preparations. In this particular case, the CPA is added into the centrifuge tubes to obtain HCDC-preparations for cryo-treatment.

Other steps, such as the cryo-treatment may be substantially similar as the steps described already in system 10A).

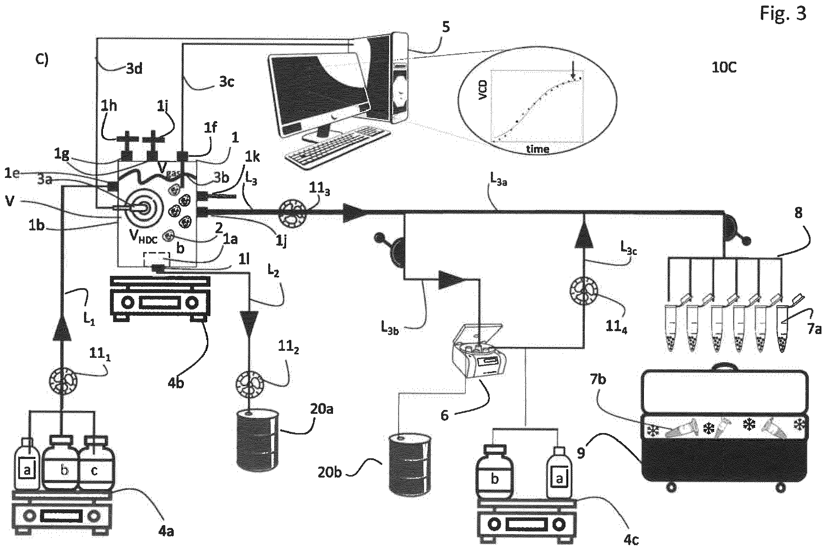

**Fig. 3** is a schematic view of a system 10C for an automated production of (cryo-preserved) HCDC-preparations according to a further embodiment. The system 10C comprises all aspects realized in system 10A and 10B, particularly the options A) and B), reflected in **Fig. 1** and **Fig. 2****,** respectively. The control unit may switch between the two options A) and B), i.e. the operation mode using the membrane element 1a or the centrifuge 6 for separating at least partially the medium b from the HCDC 2. It may even be the case that both operation modes are run parallel, for example when two different substances should be used as CPA, wherein one CPA is added using option A) and the second CPA is added using option B).

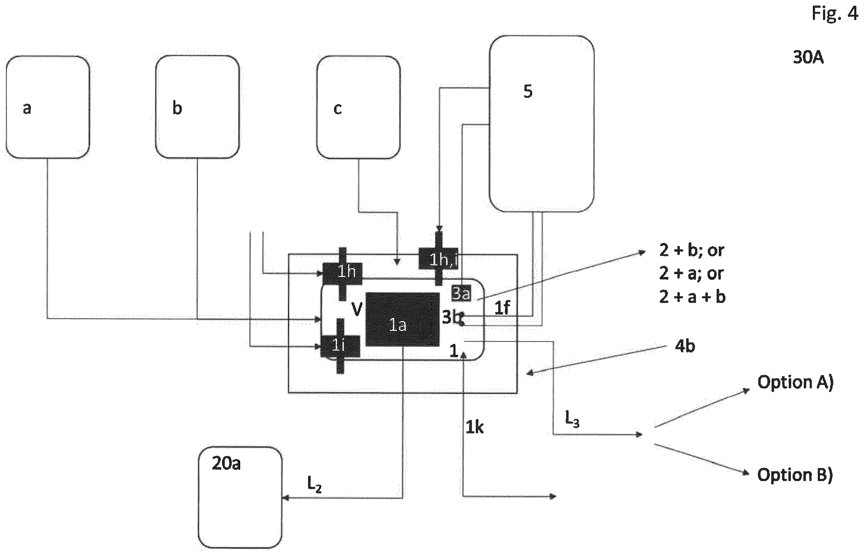

**Fig. 4** is a schematic view of a part or a section of a system 30A for an automated production of (cryo-preserved) HCDC-preparations according to an embodiment. The section of the system resembles the front portion of the above systems 10A-10C. The scheme may particularly refer to a system as it is typically used for the production of HCDC-preparations.

Herein, reservoirs of substances a, b, c, a freezing medium used as CPA (substance a), a medium (substance b) and a base and/or nutrient feed (substance c) are each fluidly connected to the device volume V. The substances a and b share a common port into the device volume V, wherein the substance c is guided by an individual port into the device volume V.

**Fig. 4** includes a top view onto the extension device 1. A membrane element 1a is arranged in the center of the extension device 1 position on the bottom of the extension device 1 right over a drain, such that the drain is covered an the cells of the HCDC is substantially retained in the device volume V when a substance such as a medium b is drained from the device volume V. In other words, the membrane element 1a, which is a membrane here, is sealed to the inner bottom of the cell extension device 1, which is a bag in the present case. A cell-free harvest line to a container 20a is substantially directly attached to the membrane element 1a. The bag 1 is positioned on a rocker 4b, which is configured for rocking the bag 1 and controlling a temperature inside the bag 1 and for agitation (the rocker being heating coild and providing a sensor).

The bag 1 typically provides four or five ports with paths or lines being fluidly connected from the device volume V, i.e. the bag volume, towards the outside.

Further, three exhaust air filters 1h,1i are arranged at or on two ports of the the bag 1 for filtering the gas, which may be air for example, which is either introduced into the device volume V or released from the device volume V. A capacitance probe 3a is arranged on the bag 1. The capacitance probe 3a may be in physical contact with the broth, comprising medium b and HCDC 2 inside the device volume V or it may be attached such that no direct physical contact is allowed. The capacitance probe 3a can transfer data to the control unit 5, also called control systems. Further sensors 3b, for example gas sensors and/or pH probes reach into the device volume V to detect parameters such as a gas concentration and/or a pH. These recorded data are also transferred to the control unit 5.

The control unit 5 is configured to control parameters comprising at least one of: a pH, a temperature, an oxygen concentration inside the device volume V, but also a rocking speed and an angle used for the rocker 4b. Further, the control unit 5 is configured to trigger a process wherein the steps of the process are based on online signals, i.e. the data related to the parameters measured over a certain period of time and being substantially instantly transferred to the control unit 5.

The port 1k may guide the broth containing HCDC 2 and medium b and/or CPA (substance a) via a path towards a tube with a dip tube, which is used for filling, for inoculation and/or for cell transfer to further processing.

The line L₃ is a cell sample line which may guide the broth towards further sections and/or units of the system either based on option A) or option B). The cell sample line may be a dip tube, i.e. a tube to the inside of the device volume V, which is used for sampling.

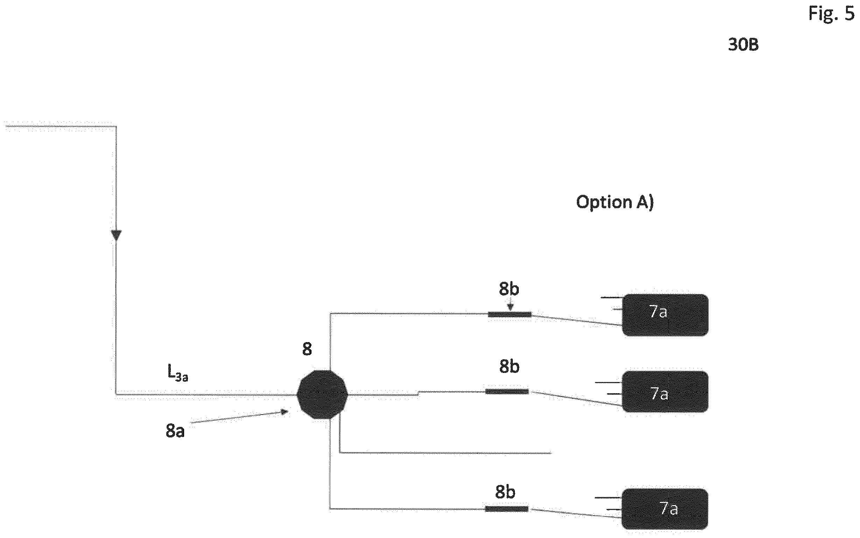

**Fig. 5** is a schematic view of a further part of a system 30B for an automated production of (cryo-preserved) HCDC-preparations according to an embodiment. The part 30B may be directly connected with the part 30A described in view of **Fig. 4****.** For example, the line L₃ which is the cell sample line may guide towards the part 30B. The part is based on the above described option A) in which the membrane element has been used to separate at least a portion of the medium from the HCDC and to replace at least a portion of the drained medium by filling the CPA into the device volume V. In other words, option A) comprises the media exchange inside the bag utilizing the in-built membrane.

A distribution unit 8 comprising a single-use manifold for even liquid distribution 8a is used to aliquot or partition the substance comprising HCDC and CPA and fill the partitioned substance into single containers, tubes 7a, and/or vessels. The distribution may be supported by the quick sealing feature 8b arranged on each line towards the tubes 7a. The parts 8, 8a, 8b and the lines may be considered the transfer unit. The distribution or transfer, comprising the filling mechanism is based on a feed-back signal, for example a bag weight.

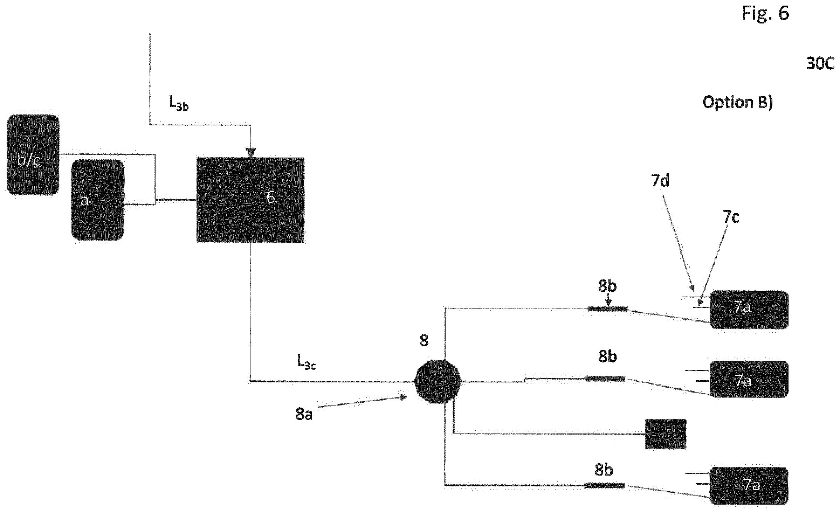

**Fig. 6** is a schematic view of a part of a system 30C for an automated production of (cryo-preserved) HCDC-preparations according to another embodiment. The part 30C may be in addition or instead to part 30B be directly connected with the part 30A described in view of **Fig. 4****.** For example, the line L₃ which is the cell sample line may guide towards the part 30C. The part is based on the above described option B) in which the centrifuge is used to separate at least a portion of the medium from the HCDC and to replace at least a portion of the separated medium by adding the CPA to the HCDC simultaneously or afterwards. In other words, option B) comprises the media exchange using centrifugation.

Basically, the part 30B described above in view of **Fig. 5** is comprised in part 30C. If both system parts based on option A) and option B), respectively are realized in one system, the part 30C may hence be shared such that a switch can switch between a bypass L₃ₐ, bypassing the centrifuge and the centrifuge. The line L_{3b} guides the broth from the device volume V into a single use centrifuge 6, particularly centrifuge containers positioned inside the centrifuge 6. A cryo medium reservoir with the substance a, i.e. CPA, as well as a reservoir for a medium b, a buffer, or the like are fluidly connected with the centrifuge container(s). Further, the centrifuge container is fluidly connected to a further container (not shown) for storing the medium which is separated from the HCDC, for example drained from the centrifuge container(s). The centrifuge is further fluidly connected by line L_{3c} with the single-use manifold 8a of the distribution unit which has already been described above in view of **Fig. 5****.** It may be possible that in a combined system based on option A) and option B), the lines Lsa and L_{3c} meet or fluidly connect substantially before or in the single-use manifold 8a. The part which is following the single-use manifold 8a may be identical to the respective part described in view of **Fig. 5****.**

The vials or tubes 7a comprise a temp-port for freezing 7c and a second line 7d.

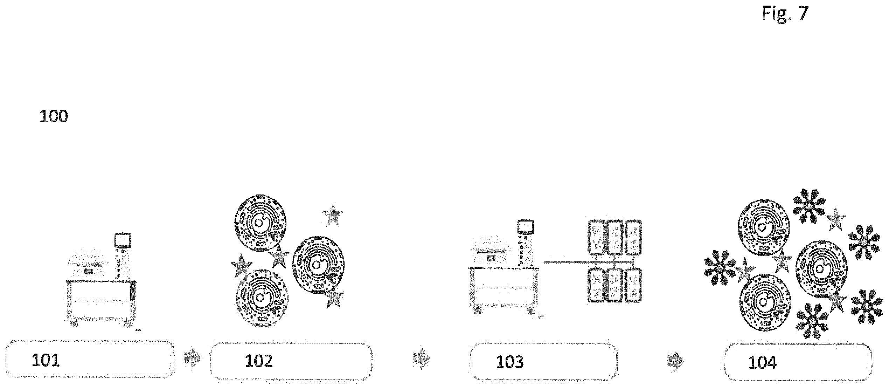

**Fig. 7** is a schematic view of the method 100 for an automated production of (cryo-preserved) HCDC-preparations. The method 100 allows producing and preparing cryo-treated, i.e. cryo-preserved HCDC-preparations, which are HCDC aliquots in this particular case. A HCDC-preparation may comprise an amount of HCDC which is substantially not partitioned, whereas a HCDC aliquot refers to one of many partitions of the HCDC-preparation. An aliquot preferably comprises a volume of approximately 1 µl to 10 ml, preferably approximately 5 µl to 1 ml and more preferably approximately 20 µl to 0,5 ml. The method 100 substantially starts with a step comprising cell expansion 101. Starter cells are combined in the device volume cell expansion device with a medium and/or a feed medium.

In a moment in time, which is well suited, for example when the viable cell density is ideal, a step is performed in which the CPA is added 102 to the HCDC. This step may comprise removal, particularly draining the medium through the drain and the membrane while retaining the HCDC inside the device volume. The CPA may at least partially replace the volume of the drained medium. Preferably, the volume of the drained medium is substantially replaced by addition of the CPA, as in this case, the cell concentration, particularly the viable cell concentration can be maintained stable. Alternatively, or in addition, the step 102 may comprise the centrifugation of the HCDC with the medium, the physical separation of the HCDC from a least a portion of the medium, for example by draining and/or sucking out the medium, and the addition of CPA to the HCDC. Also in this case, the volume of the drained or sucked out medium is preferably substantially replaced by addition of the CPA, as in this case, the cell concentration, particularly the viable cell concentration can be maintained stable.

Particularly referring to option A), in the case of using a membrane element to drain at least partially the medium, the addition of CPA 102 is followed by the step of transferring the HCDC with the CPA into vials and/or containers for cryo-storage. In the present method of **Fig. 7****,** the step of transferring comprises aliquoting 103 the HCDC with the CPA into smaller volume portions of the generated volume of the HCDC with the CPA. In other words, the volume which has been generated and prepared is partitioned into smaller volumes and transferred, for example filled into vials, (storage) containers, and/or tubes, such as polymer tubes suited for freezing.

Alternatively, the step of aliquoting may already be performed before the addition of CPA, particularly referring to option B). In this case, HCDC and medium is transferred, particularly aliquoted into centrifuge containers which are used for centrifugation and for cryo-treatment. The CPA may be added into the centrifuge containers after the step of aliquoting.

However, even in the case of option B) the aliquotation 103 or a second aliquotation may be performed after the addition of CPA, particularly when the HCDC with CPA is transferred from the centrifuge container(s) into receiving containers.

The aliquoted HCDC with CPA in vials, vessels, containers and/or tubes can be considered HCDC-preparations, particularly HCDC-aliquots for cryo-treatment. The step of cryo-treatment comprising the step of freezing 104 the HCDC-aliquots is the last step of the exemplary method described in **Fig. 7****.**

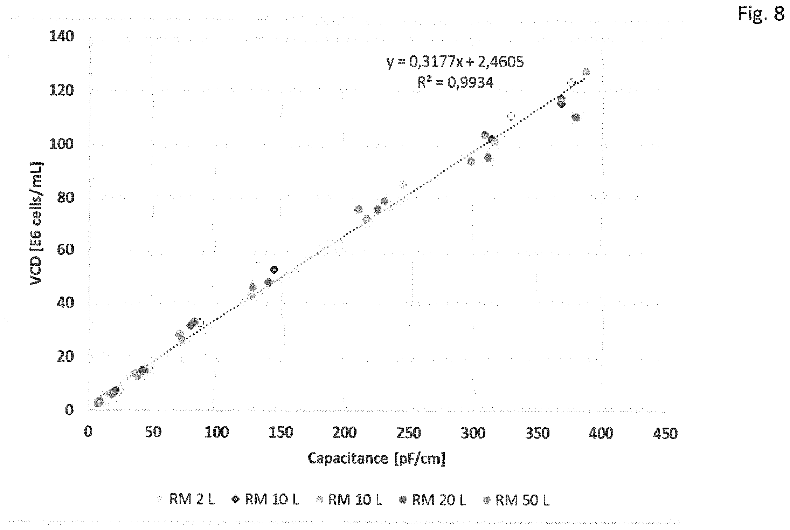

**Fig. 8** is an exemplary plot of viable cell concentration depending plotted against the measured capacitance. In the present example the at least one sensor for collecting data comprises an electrode sensor, particularly a capacitance probe or capacitance sensor. The capacitance probe at least temporally monitors at least one parameter, i.e. the capacitance of the substance inside the device volume comprising the HCDC. The parameter is indicative of a physiological status of the HCDC, as the viable cell density indicates a linear correlation with the increase of a measured capacitance at least in the range of the illustrated capacitance (approximately 5 to 400 pF/cm). The linear correlation is indicated by the dotted line. In other words, the measured capacitance is a direct linear indicator for the viable cell density (VDC), wherein the VDC is considered a dimension also describing the physiological status of the HCDC.

In perfusion processes there is a very good correlation between viable cell density and online signals like capacitance as the cells do not swell too much compared to standard fed-batch processes. The plot of **Fig. 8** refers to the viable cell density (VCD)-capacitance correlation for CHO cells for different RM bag sizes.

Capacitance probes, like the BioPAT ViaMass, are very suitable to monitor the viable cell densities online without physical interaction with the bag. This sensor platform allows automatic determination of the ideal time-point for further processing of the culture, alternative sensor solutions (amongst others) would be Raman spectrometry. In addition to this, additional and complementing sensors do help to determine substrate or product concentration online. Inhibitory process conditions like nutrient depletion can be prevented by keeping nutrient level at ideal conditions. Furthermore cell specific consumption or production rates as well as specific growth rates can be determined (in combination with the viable cell density read-out) and serve as an indicator for cellular state. For example, Raman spectrometry and enzyme-based online sensors (e.g. BioPAT Trace) would be capable to provide the relevant substrate and product concentrations.

In the following definitions which are used herein and exemplary further descriptions for better understanding the invention are given:
The term "High Cell Density Seed Cultures" (HCDCs) refers to cell cultures having a high density of cells particularly viable cells, i.e. a volume concentration of cells which are at least alive. Preferably, HCDCs comprise at least approximately 5 Mio. cells per mL (5 x 10⁶ cells/mL), preferably at least approximately 20 Mio. cells per mL (20 x 10⁶ cells/mL) and more preferably at least approximately 100 to 120 Mio. cells per mL (20 x 10⁶ cells/mL), wherein the cells are preferably viable. The term may however also refer to the very beginning of the growth of a HCDC, i.e. when for example only a couple of hundreds or thousands of viable cells per mL start growing to cultivate a HCDC in the above sense. The term HCDC as used herein may hence also refer to a cell culture which is any state of its growth being grown for the purpose of having a high density of viable cells in the above sense.

The term "cell culture" preferably refers to the process by which cells are grown under controlled conditions, generally outside their natural environment, i.e. *ex situ.* After the cells are isolated from a living tissue, they can subsequently be maintained under carefully controlled conditions. Such conditions can vary for each cell type, but generally they may depend on a suitable vessel, for example a cell expansion device, with a substrate or medium that supplies the essential nutrients particularly, amino acids, carbohydrates, vitamins and/or minerals. Further, the conditions may relate to growth factors, hormones, and gases, such as CO₂ and O₂, and the physio-chemical environment, particularly the pH buffer, the osmotic pressure, and/or the temperature.

Some cells require a surface or an artificial substrate (particularly an adherent or a monolayer culture) whereas others can be grown free floating in culture medium (suspension culture). Preferably, cells are referred to herein which can be grown free floating in a culture medium, which is referred to a "a medium". The lifetime of most types of cells is genetically determined, but some cell culturing cells have been "transformed" into immortal cells which will reproduce indefinitely if the optimal conditions are provided. The lifetime and reproduction rate of cells may hence vary.

The "HCDC-preparation" as described herein is considered a product which is substantially ready for cryo-treatment and which is generated by one of the systems and the method according to any one aspect. The "HCDC-preparation" is preferably in a continuous operation. It is also possible, that the HCDC-preparation is generated in a batch operation or in a fed-batch operation. In general, a distinction can be made between:
- Batch operation: filling of the reactor with cells and substances, no addition or removal during the cell extension process, substantially external control of parameters, contamination is unlikely;
- Fed-batch operation: similar to batch operation, however, for example a selected substrate may be added during the cell extension process;
- Continuous operation: substrate addition and product removal cell extension process, control of parameters, contamination cannot be excluded.

A HCDC preparation which is substantially ready for the cryo-treatment, preferably is a substance comprising HCDC and CPA. The substance may also comprise residual portions of the medium in which the cells have grown. At least a portion of the substance volume is stored in one or more receiving devices. Preferably, before cryo-treatment, the substance volume is partitioned into multiple portions of small volumes being filled into tubes. The substance volume is substantially liquid before the cryo-treatment and may substantially be solid after the cryo-treatment. The freezing point, i.e. the temperature at which the substance may become substantially solid may deviate from the freezing point of water, i.e. approximately 0°C under normal conditions.

A "cryo-preserved HCDC-preparation" is referred to as a HCDC preparation after cryo-treatment, i.e. a HCDC-preparation which is cooled down to a certain temperature, particularly frozen.

The term "cryo-treatment" which is substantially the same as "cryo-preservation" refers to a procedure of cooling down, particularly freezing the substance which comprises HCDC and CPA. The procedure may be a step-wise freezing of the substance. For example, the substance may be cooled down from a room temperature to approximately 4°C (typical fridge temperature) and/or to approximately -6 to -20°C (typical freezer temperature) and/or to approximately -78,5°C (sublimation point of dry ice) and/or to approximately -196 °C (boiling point of liquid nitrogen). The cryo-treatment may also refer to a sudden step of cooling the substance down to a certain temperature, for example from approximately room temperature down to approximately -196 °C. Before the cryo-treatment any other step that can be required or advantageous to prepare the substance for the cryo-treatment, such as the addition of other compounds, are possible.

Small portions of the volume of substance comprising HCDC and CPA, and possibly the medium and/or other compounds is referred to as aliquots or HCDC aliquots, herein.

Monitoring a process particularly comprises detecting, recording and potentially but not necessarily displaying of at least one parameter of a process, particularly a parameter which is indicative of the state of the process. Monitoring also refers to at least temporally collecting a set of data. The monitoring may be performed by a continuous data collection over a period of time or by a discrete data collection, i.e. a collection of data only at discrete points in time.

The term "online monitoring" particularly refers to detecting at least one parameter which is indicative of the state of a bio process substantially in real time, particularly the physiological state of the cell culture, herein the HCDC. In other words, a process permanently changes the state of a HCDC. With online monitoring, at least one parameter is detected, i.e. measured at least once but preferably it is detected over a period of time or at least a couple of distinct times. The measurement is substantially instantaneous, i.e. the measurement result indicating an instantaneous state can be obtained substantially at the same time, i.e. in a moment in which the state has not substantially changed.

The term "viable cell density" particularly refers to the density of substantially viable cells per volume.

The term "sensor" particularly refers to a detector and/or a probe configured to sense, probe or detect at least one parameter or physical dimension, quantity, variable or value.

The term "a membrane element arranged with the drain" particularly refers to an arrangement of the membrane element inside the device volume and in front of the drain, for example covering the drain such that the HCDC inside the device volume is retained when at least a portion of the medium is drained from the device volume through the membrane element. The membrane element may however also be arranged inside the device volume to partition the device volume into two device volume compartments, a first and a second compartment, wherein the drain is arranged such that a fluid connection between the outside and the inside of one of both compartments can be generated when the drain is opened, for example to drain a substance from the first compartment through the drain. In that particular case, also a substance, for example a liquid from the second compartment may be drained if this substance can diffuse through the membrane element.

A cryo-protective agent (CPA), usually also referred to as cryoprotectant, preferably comprises at least any one of: dimethyl sulfoxide (DMSO), glycerol, ethylene glycol, terahalose, sucrose, propylene glycol and 2-Methyl-2,4-pentanediol (MPD). The CPA protects the cells during a cryo-treatment from denaturation, such that a high amount of the cryo-preserved cells can be used as substantially viable cells after defrosting.

A medium, which is considered a growth medium herein, preferably comprises a carbon source such as Glucose, water, various salts, particularly a buffer medium, a source of amino acids and nitrogen. A feed medium may for example comprise similar substances, wherein the nutrition may be present at higher concentrations. The medium is considered a substantially liquid medium, herein. All substances which support the growth of the cells or which have a function related to the physiological condition or state of the HCDC may be comprised in a medium and/or a feed medium. Alternatively, also a buffer substantially comprising only ions, i.e. salt and water, may be considered a medium. A medium may even be considered water without any compounds.

The phrase "at least one predetermined criterion is fulfilled by the monitored parameter" refers to a setting that is chosen by an operator for example. The operator preferably choses a threshold for the viable cell density, at which a process step is initiated. For example, when the HCDC reaches a viable cell density threshold of approximately 20 x 10⁶ viable cells /ml, a fresh feed medium may be introduced into the device volume. For example, when the HCDC reaches a viable cell density threshold of approximately 80 x 10⁶ viable cells /ml, a fresh medium may be introduced into the device volume. For example, when the HCDC reaches a viable cell density threshold of approximately 100 x 10⁶ viable cells /ml, a CPA may be introduced into the device volume while or after a substance, such as a portion of the medium is drained from the device volume. Alternatively or in addition, a function according to a theory (for example, a theory of exponential growth) is fitted automatically by the control unit to the viable cell density as a function of time. Instead or in addition of a threshold, the at least one predetermined criterion may also comprise a value for a derivative of the fitted function.

A receiving unit is preferably a bag, a vial, a tube or a container. Transfer units preferably comprise units for clipping, sterile adapters and/or sealing units. Cryo-units particularly comprise controlled rate-free freezing units or non-controlled-rate freezing. A transfer unit preferably comprises at least one out of a distribution unit 8, and paths guiding a substance out of the device volume L3a, L3b and/or towards a distribution unit L3a, L3c.

After the cryo-treatment, the cryo-preserved HCDC-preparations can be transferred for a mid-term storage or a long-term storage being available to the end user on demand.

Device volumes V and volumes of substances inside the device volume V preferably range between approximately 0,5 I and 150 I, preferably between approximately 0,75 I and 50 I, and more preferably between approximately 11 and 25 I.

Flow control devices preferably comprise at least one out of: a flowmeter, a balance, a calibrated pump.

Liquid transfer devices preferably comprise at least one out of: a pump, pneumatic, gravimetric, a balance, pressure driven units.

Distribution units preferably comprise at least one out of: a multi-way valve, a manifold. To better understand the invention, the methods and systems according to several aspects are described below, noting that these methods and systems do not correspond to the claimed invention:
Substantial exemplary steps for the generation of frozen HCDC seed cultures may be
1. Cell expansion comprising the growth of a HCDC to generate sufficient amounts of cells per volume
2. Applying a cryo-protective agent, CPA, to the cells to prepare or preserve them for freezing
3. Aliquoting defined cell volumes into vessel for freezing, i.e. partitioning a produced volume of HCDC
4. Freezing of cells

A system for the generation of HCDC frozen seed cultures may comprise
- a cell expansion device including an in-built membrane-based cell retention device
- process liquids
- a sensor or combinations thereof to monitor the physiological status of the cells as well as the viable cell density
- pumps
- balances or other means of volume control
- a control unit to control the cultivation, the transfer of volumes between process steps and to initiate the preparation of cells for freezing
- liquid transfer devices
- flow control devices
- flow distribution device
- receiving devices for freezing the cells
- Further optional equipment may be
- tempering device to cool the liquid to allow a safe treatment of cells
- in-line mixing device for quick and sufficient mixing of cells with cryo-protective agent
- a cell separation device if cell separation is done outside the cell expansion device

A system for the generation of high cell density cryo-cultures, may comprise particularly
- at least one cell expansion device, including an in-built, membrane-based cell retention device
- process liquids, such as a medium, pH corrective, and/or a CPA solution
- a sensor or combinations thereof, able to provide online information on the physiological status of cells and viable cell density
- pumps
- balances or equivalent means of volume control for feed and harvest
- a control unit
   - to control the cultivation
   - to automatically monitor the culture state and to initiate the culture preparation for freezing based on the sensor input(s)
   - to control the transfer of volumes between the different process steps
- an optional tempering device
- an optional inline mixing device
- liquid transfer device
- a flow control device
- a flow distribution device
- and a plurality of receiving devices

A system for the generation of high cell density cryo-cultures, may also comprise
- at least one cell expansion device, including an in-built, membrane-based cell retention device
- process liquids, such as medium, pH corrective, CPA solution]
- a sensor or combinations thereof, able to provide online information on the physiological status of cells and viable cell density
- pumps
- balances or equivalent means of volume control for feed and harvest
- a control unit
   - to control the cultivation
   - to automatically monitor the culture state and to initiate the culture preparation for freezing based on the sensor input(s)
   - to control the transfer of volumes between the different process steps
- cell separation device and intermediate storage
- liquid transfer device
- a flow control device
- a flow distribution device
- and a plurality of receiving devices

A method for the generation of high cell density cryo-cultures, may also comprise
- cultivating suspension cells in at least one cell expansion device,
   - having working volumes of 0.1 - 25 L
   - including an in-built , membrane-based cell retention device
   - viable cell densities of 100 E6 viable cells/mL and more
   - process mode: perfusion
- process liquid, such as medium, pH corrective, CPA solution
- using a sensor or combinations thereof, able to provide information on the physiological status of cells and viable cell density
- pumps
- balances or equivalent means of volume control for feed and harvest
- a control unit
   - to control the cultivation
   - to automatically monitor the culture state and to initiate the culture preparation for freezing based on the sensor input(s)
   - to control the transfer of volumes between the different process steps
- a step to replace a part of or the entire the culture volume with CPA-containing medium
   - e.g. by removing cell-free supernatant using the in-built membrane device and topping it up with CPA-solution
   - e.g. by removing cell-free supernatant using the in-built membrane device and performing in-line mixing with CPA-solution upon transfer to next process step using a mixing device in the transfer line
- a step to reduce the temperature of the cell containing solution, e.g. by using a fast tempering device or pre-cooled medium
- liquid transfer device to transfer the CPA-containing cell suspension, such as pumps, pneumatic, gravimetric and/or pressure-driven
- a flow control device
- a flow distribution device
   - multi-valve or manifold-based
- and a plurality of receiving devices
   - vials, bags, and/or vessels with a volume of not more than 100 mL

A method for the generation of high cell density cryo-cultures, may also comprise
- cultivating suspension cells in at least one cell expansion device,
   - having working volumes of approximately 0,1 - 25 L
   - including an in-built , membrane-based cell retention device
   - viable cell densities of 100 E6 viable cells/mL and more
   - process mode: Perfusion
- process liquid
- using a sensor or combinations thereof, able to provide information on the physiological status of cells and viable cell density
- pumps
- balances or equivalent means of volume control for feed and harvest
- a control unit
   - to control the cultivation
   - to automatically monitor the culture state and to initiate the culture preparation for freezing based on the sensor input(s)
   - to control the transfer of volumes between the different process steps
- a step to replace a part of or the entire the culture volume with CPA-containing medium
   - by using a single-use cell separation device to replace the culture medium with pre-tempered CPA-containing solution followed by a subsequent intermediate storage
      - preferred embodiment: single-use centrifuge
- a step to reduce the temperature of the cell containing solution, e.g. by using a fast tempering device or pre-cooled medium
- liquid transfer device to transfer the CPA-containing cell suspension
- a flow control device
- a flow distribution device
   - multi-valve or manifold-based
- and a plurality of receiving devices
   - vials/bags/vessels with a volume of not more than 100 mL or less than 100 mL

### Reference Signs

| | |
|---|---|
| 1 | Cell expansion device |
| 1a | Membrane element |
| 1b | Skin of the cell expansion device |
| 1c | Top ceiling of the cell expansion device |
| 1d | Bottom of the cell expansion device |
| 1e | At least one port for substance supply |
| 1f | Sensor port |
| 1g | Ports used as inlet and/or outlet |
| 1h | Filter element |
| 1i | Filter element |
| 1i | Port for cell sample line |
| 1k | Port |
| 1l | Drain, port for cell-free harvest |
| 2 | High cell density seed culture |
| 3a | Electrode sensor, particularly capacitance sensor |
| 3b | Sensor |
| 3c | Data transfer unit of sensor |
| 3d | Data transfer unit of electrode sensor |
| 4a | Unit with rocker and/or balance and/or magnetic stirring and/or temperature regulating function |
| 4b | Unit with rocker and/or balance and/or magnetic stirring and/or temperature regulating function |
| 4c | Unit with rocker and/or balance and/or magnetic stirring and/or temperature regulating function |
| 5 | Control unit |
| 6 | (single use centrifuge |
| 7a | Tube |
| 7b | Cryo-preserved HCDC preparations |
| 7c | Temp-port for freezing |
| 7d | Second line |
| 8 | Distribution unit |
| 8a | Single-use manifold for even liquid distribution |
| 8b | Quick sealing feature |
| 9 | Cryo-Unit |
| 10A | System A |
| 10B | System B |
| 10C | System C: Combination of System A and System B |
| 11₁ | First flow control unit |
| 11₂ | Second flow control unit |
| 11₃ | Third flow control unit |
| a | Cryo-protective agent, CPA |
| b | Liquid culture medium |
| c | Base or nutrition feed/ feed medium |
| L₁, L₂, L₃, L₃ₐ, L_{3b}, L_{3c} | Path |
| V | Device Volume |
| V_{HCDC} | Volume of the HCDC |
| V_{gas} | Volume of the gas |
| 100 | Method comprising the steps 101-104 |
| 101 | Step comprising cell expansion |
| 102 | Step comprising the addition of CPA |
| 103 | Step comprising aliquoting |
| 104 | Step comprising cryo-treatment |

## Claims

1. System (10A; 10B; 10C) for an automated production of high cell density seed culture, HCDC, preparations, for cryo-preservation comprising:
- a cell expansion device (1) for growing a HCDC (2) in a medium (b) inside a device volume (V) of the cell expansion device (1),
- at least one sensor (3a, 3b) for collecting data by at least temporally monitoring at least one parameter being indicative of the physiological state of the HCDC; and
- a control unit (5) for processing the collected data to determine whether at least one predetermined criterion is fulfilled by the monitored parameter to control, based on whether the at least one predetermined criterion is fulfilled by the monitored parameter, at least a partial exchange of the medium (b) by a cryo-protective agent, CPA (a);
wherein the cell expansion device (1) comprises
- at least one drain (1l) for draining at least a portion of the medium (b) from the device volume (V) towards the outside of the device volume (V); and
- a membrane element (1a) arranged at the drain (1l) such that the HCDC (2) is retained inside the device volume (V) when at least a portion of the medium (b) is drained from the device volume (V) through the membrane element (1a) and through the drain (1l) to the outside of the device volume (V); and
wherein the control unit (5) is configured to initiate, based on whether or not the criterion is fulfilled, the steps of
- draining at least a portion of the medium (b) inside the device volume (V) through the membrane element (1a); and,
- adding the CPA (a) to the HCDC (2) in the device volume (V), and
wherein the system comprises a transfer unit (8; L3a; L3b, L3c), controlled by the control unit (5), for transferring at least a portion of the HCDC (2) with CPA (a) from the device volume (V) into one or more receiving devices (7a) to obtain HCDC-preparations for cryo-preservation; and
wherein the system (10A; 10B; 10C) further comprises:
- a centrifuge unit (6) for centrifuging the HCDC (2) with the medium (b);
- at least one transfer unit, controlled by the control unit (5), for transferring at least a portion of the HCDC (2) with the medium (b) into at least one centrifuge container of the centrifuge unit (6); and
wherein the control unit (5) is configured to initiate, based on whether or not the criterion is fulfilled, the steps of transferring at least a portion of the HCDC (2) with the medium (b) from the device volume (V) into the at least one centrifuge container of the centrifuge unit (6), centrifuging with the centrifuge unit (6) the transferred HCDC (2) with the medium (b), separating at least partially the centrifuged medium (b) from the HCDC (2), and adding a cryo-protective agent, CPA (a), to the HCDC (2).

2. The system of claim 1, wherein the transfer unit (8; L3a; L3b, L3c) is configured to automatically transfer at least a portion of the HCDC (2) with CPA (a) from the device volume (V) into one or more receiving devices (7a) to obtain HCDC-preparations for cryo-preservation.

3. The system (10A; 10B; 10C) of any one of the preceding claims, wherein the sensor (3a, 3b) forms an integral unit with the cell expansion device (1), preferably, wherein the sensor (3a, 3b) is welded into at least a portion of a wall (1b) of the cell expansion device (1) and/or wherein the sensor (3a, 3b) inserts into the device volume (V) through a port of the cell expansion device (1).

4. The system (10A; 10C) of any one of claims 1 to 3, wherein the membrane element (1a) forms an integral unit with the cell expansion device (1).

5. The system (10A; 10B; 10C) of any one of the preceding claims, wherein the control unit (5) is configured to, based on the monitored parameter, automatically control at least one second parameter, wherein the second parameter is preferably configured to control and/or regulate the physiological status of the HCDC.

6. The system (10A; 10B; 10C) of any one of the preceding claims, wherein at least one out of the cell expansion device (1), the sensor (3a, 3b), the centrifuge unit (6), the receiving devices comprising a bag, a tube and/or a vial, and at least a portion of the transfer unit (8; L3a; L3b, L3c) is a single-use element and/or a single use consumable.

7. Method for an automated production of high cell density seed culture preparations, HCDC-preparations, for cryo-preservation, comprising the steps of
- growing a HCDC (2) in a medium (b) inside a device volume (V) of the cell expansion device (1);
- collecting data by at least temporally monitoring at least one parameter wherein the parameter is indicative of a physiological status of the HCDC (2);
- sending the collected data to a control unit (5);
- processing by the control unit (5) the collected data at least comprising determining whether at least one predetermined criterion is fulfilled by the monitored parameter; and
- controlling, based on whether the at least one predetermined criterion is fulfilled by the monitored parameter, at least a partial exchange of the medium (b) by a cryo-protective agent, CPA (a);
wherein, based on whether or not the monitored parameter fulfills the at least one criterion:
- initiating by the control unit (5) the steps comprising
Option A)
draining via a membrane element (1a) for retaining the HCDC (2) inside the device volume (V) and via a drain (11) at least a portion of the medium (b) from the device volume (V),
adding a cryo-protective agent, CPA (a), to the HCDC (2) in the device volume (V), and
transferring at least a portion of the HCDC (2) in the CPA (a) from the device volume (V) into one or more receiving devices (7a) to obtain HCDC-preparations for cryo-preservation, wherein the step of transferring is performed automatically; and/or
Option B)
transferring at least a portion of the HCDC (2) with the medium (b) from the device volume (V) into at least one centrifuge container of the the centrifuge unit (6),
centrifuging with the centrifuge unit (6) the transferred HCDC (2) with the medium (b),
separating at least partially the centrifuged medium (b) from the HCDC (2), and
adding CPA (a) to the HCDC (2) to obtain HCDC-preparations for cryo-preservation, wherein transferring at least a portion of the HCDC (2) with the CPA (a) from the centrifuge container into one or more receiving devices (7a) to obtain HCDC-preparations for cryo-preservation, wherein the step of transferring at least a portion of the HCDC (2) with the medium (b) from the device volume (V) into at least one centrifuge container and/or transferring at least a portion of the HCDC (2) with the CPA (a) from the centrifuge container into one or more receiving devices (7a) is performed automatically.

8. The method of claim 7, further comprising, based on the monitored parameter, automated controlling, by the control unit (5), of at least one second parameter, wherein the second parameter is configured for controlling and/or regulating a physiological status of the HCDC (2).

9. The method of any one of claims 7 or 8, wherein the processing of the collected data comprises automatically analyzing the collected data by fitting a function and/or model building to at least a portion of the collected data to obtain a fit result.

10. The method of claim 9, wherein the determining whether at least one predetermined criterion is fulfilled by the monitored parameter is based on automatically analyzing of the fit result.

11. The method of any one of claims 7 to 10, further comprising the step of automatically cryo-preserving the HCDC-preparations in the one or more receiving devices (7a) to obtain cryo-preserved HCDC-preparations (2).

12. The method of any one of claims 7 to 11, further comprising, in at least one iteration step, determining the quality of the obtained HCDC-preparations and
- varying the predetermined criterion to be fulfilled by the monitored parameter and/or
- varying at least one second parameter; and
- repeating the method of any one of claims 8 to 13, particularly until an optimal quality is obtained.

## Patentansprüche

1. System (10A; 10B; 10C) für eine automatisierte Produktion von Zubereitungen einer Saatkultur mit hoher Zelldichte (High Cell Density Seed Culture), HCDC, zur Kryokonservierung, umfassend:
- eine Zellexpansionsvorrichtung (1) zum Züchten einer HCDC (2) in einem Medium (b) im Inneren eines Vorrichtungsvolumens (V) der Zellexpansionsvorrichtung (1),
- mindestens einen Sensor (3a, 3b) zum Sammeln von Daten durch mindestens zeitweiliges Überwachen mindestens eines Parameters, der auf den physiologischen Zustand der HCDC hindeutet; und
- eine Steuereinheit (5) zum Verarbeiten der gesammelten Daten, um zu bestimmen, ob mindestens ein vorbestimmtes Kriterium von dem zu überwachenden Parameter erfüllt ist, um basierend darauf, ob das mindestens eine vorbestimmte Kriterium von dem überwachten Parameter erfüllt ist, mindestens einen partiellen Austausch des Mediums (b) mit einem Kryoschutzmittel (Cryo-Protective Agent), CPA, (a) zu steuern;
wobei die Zellexpansionsvorrichtung (1) umfasst:
- mindestens einen Ablass (11) zum Ablassen mindestens eines Teils des Mediums (b) von dem Vorrichtungsvolumen (V) hin zur Außenseite des Vorrichtungsvolumens (V); und
- ein Membranelement (1a), das derart an dem Ablass (11) angeordnet ist, dass die HCDC (2) im Inneren des Vorrichtungsvolumens (V) zurückgehalten wird, wenn mindestens ein Teil des Mediums (b) von dem Vorrichtungsvolumen (V) durch das Membranelement (1a) und durch den Ablass (11) zur Außenseite des Vorrichtungsvolumens (V) abgelassen wird; und
wobei die Steuereinheit (5) dazu ausgestaltet ist, basierend darauf, ob das Kriterium erfüllt ist oder nicht, die folgenden Schritte einzuleiten:
- Ablassen mindestens eines Teils des Mediums (b) im Inneren des Vorrichtungsvolumens (V) durch das Membranelement (1a); und
- Hinzufügen des CPA (a) zu der HCDC (2) in dem Vorrichtungsvolumen (V), und
wobei das System eine Transfereinheit (8; L3a; L3b, L3c), die von der Steuereinheit (5) gesteuert wird, zum Transferieren mindestens eines Teils der HCDC (2) mit CPA (a) von dem Vorrichtungsvolumen (V) in eine oder mehrere Aufnahmevorrichtungen (7a) umfasst, um HCDC-Zubereitungen zur Kryokonservierung zu erhalten; und
wobei das System (10A; 10B; 10C) ferner umfasst:
- eine Zentrifugeneinheit (6) zum Zentrifugieren der HCDC (2) mit dem Medium (b);
- mindestens eine Transfereinheit, die von der Steuereinheit (5) gesteuert wird, zum Transferieren mindestens eines Teils der HCDC (2) mit dem Medium (b) in mindestens einen Zentrifugenbehälter der Zentrifugeneinheit (6); und
wobei die Steuereinheit (5) dazu ausgestaltet ist, basierend darauf, ob das Kriterium erfüllt ist oder nicht, die Schritte des Transferierens mindestens eines Teils der HCDC (2) mit dem Medium (b) von dem Vorrichtungsvolumen (V) in den mindestens einen Zentrifugenbehälter der Zentrifugeneinheit (6), des Zentrifugierens der transferierten HCDC (2) mit dem Medium (b) mit der Zentrifugeneinheit (6), des mindestens partiellen Trennens des zentrifugierten Mediums (b) von der HCDC (2) und des Hinzufügens eines Kryoschutzmittels, CPA, (a) zu der HCDC (2) einzuleiten.

2. System nach Anspruch 1, wobei die Transfereinheit (8; L3a; L3b, L3c) dazu ausgestaltet ist, mindestens einen Teil der HCDC (2) mit CPA (a) automatisch von dem Vorrichtungsvolumen (V) in eine oder mehrere Aufnahmevorrichtungen (7a) zu transferieren, um HCDC-Zubereitungen zur Kryokonservierung zu erhalten.

3. System (10A; 10B; 10C) nach einem der vorhergehenden Ansprüche, wobei der Sensor (3a, 3b) eine einteilige Einheit mit der Zellexpansionsvorrichtung (1) bildet, wobei der Sensor (3a, 3b) vorzugsweise in mindestens einen Abschnitt einer Wand (1b) der Zellexpansionsvorrichtung (1) geschweißt ist und/oder wobei der Sensor (3a, 3b) sich durch eine Öffnung der Zellexpansionsvorrichtung (1) in das Vorrichtungsvolumen (V) einsetzt.

4. System (10A; 10C) nach einem der Ansprüche 1 bis 3, wobei das Membranelement (1a) eine einteilige Einheit mit der Zellexpansionsvorrichtung (1) bildet.

5. System (10A; 10B; 10C) nach einem der vorhergehenden Ansprüche, wobei die Steuereinheit (5) dazu ausgestaltet ist, basierend auf dem überwachten Parameter mindestens einen zweiten Parameter automatisch zu steuern, wobei der zweite Parameter vorzugsweise dazu ausgestaltet ist, den physiologischen Zustand der HCDC zu steuern und/oder zu regeln.

6. System (10A; 10B; 10C) nach einem der vorhergehenden Ansprüche, wobei mindestens eines von der Zellexpansionsvorrichtung (1), dem Sensor (3a, 3b), der Zentrifugeneinheit (6) und den Aufnahmevorrichtungen einen Beutel, einen Schlauch und/oder ein Fläschchen umfasst und mindestens ein Teil der Transfereinheit (8; L3a; L3b, L3c) ein Einwegelement und/oder ein Einwegverbrauchsmaterial ist.

7. Verfahren zur automatisierten Herstellung von Zubereitungen von Saatkulturen mit hoher Zelldichte, HCDC-Zubereitungen, zur Kryokonservierung, das die folgenden Schritte umfasst:
- Züchten einer HCDC (2) in einem Medium (b) im Inneren eines Vorrichtungsvolumens (V) der Zellexpansionsvorrichtung (1);
- Sammeln von Daten durch mindestens zeitweiliges Überwachen mindestens eines Parameters, wobei der Parameter auf einen physiologischen Zustand der HCDC (2) hindeutet;
- Senden der gesammelten Daten an eine Steuereinheit (5);
- Verarbeiten der gesammelten Daten durch die Steuereinheit (5), das mindestens das Bestimmen, ob mindestens ein vorbestimmtes Kriterium von dem überwachten Parameter erfüllt wird, umfasst; und
- Steuern mindestens eines partiellen Austauschs des Mediums (b) mit einem Kryoschutzmittel, CPA, (a), basierend darauf, ob das mindestens eine vorbestimmte Kriterium von dem überwachten Parameter erfüllt wird;
wobei, basierend darauf, ob der überwachte Parameter das mindestens eine Kriterium erfüllt oder nicht:
- Einleiten, durch die Steuereinheit (5), der Schritte, die umfassen:
Option A)
Ablassen, über ein Membranelement (1a) zum Zurückhalten der HCDC (2) im Inneren des Vorrichtungsvolumens (V) und über einen Ablass (11), mindestens eines Teils des Mediums (b) von dem Vorrichtungsvolumen (V),
Hinzufügen eines Kryoschutzmittels, CPA, (a) zu der HCDC (2) in dem Vorrichtungsvolumen (V), und
Transferieren mindestens eines Teils der HCDC (2) in dem CPA (a) von dem Vorrichtungsvolumen (V) in eine oder mehrere Aufnahmevorrichtungen (7a), um HCDC-Zubereitungen zur Kryokonservierung zu erhalten, wobei der Schritt des Transferierens automatisch durchgeführt wird; und/oder
Option B)
Transferieren mindestens eines Teils der HCDC (2) mit dem Medium (b) von dem Vorrichtungsvolumen (V) in mindestens einen Zentrifugenbehälter der Zentrifugeneinheit (6),
Zentrifugieren, mit der Zentrifugeneinheit (6), der transferierten HCDC (2) mit dem Medium (b),
mindestens partielles Trennen des zentrifugierten Mediums (b) von der HCDC (2), und
Hinzufügen von CPA (a) zu der HCDC (2), um HCDC-Zubereitungen zur Kryokonservierung zu erhalten, wobei das Transferieren mindestens eines Teils der HCDC (2) mit dem CPA (a) von dem Zentrifugenbehälter in eine oder mehrere Aufnahmevorrichtungen (7a), um HCDC-Zubereitungen zur Kryokonservierung zu erhalten, wobei der Schritt des Transferierens mindestens eines Teils der HCDC (2) mit dem Medium (b) von dem Vorrichtungsvolumen (V) in mindestens einen Zentrifugenbehälter und/oder des Transferierens mindestens eines Teils der HCDC (2) mit dem CPA (a) von dem Zentrifugenbehälter in eine oder mehrere Aufnahmevorrichtungen (7a) automatisch durchgeführt wird.

8. Verfahren nach Anspruch 7, das ferner basierend auf dem überwachten Parameter das automatisierte Steuern, durch die Steuereinheit (5), mindestens eines zweiten Parameters umfasst, wobei der zweite Parameter dazu ausgestaltet ist, einen physiologischen Zustand der HCDC (2) zu steuern und/oder zu regeln.

9. Verfahren nach einem der Ansprüche 7 oder 8, wobei das Verarbeiten der gesammelten Daten das automatische Analysieren der gesammelten Daten durch Anpassen einer Funktion und/oder Modellbildung an mindestens einen Teil der gesammelten Daten umfasst, um ein Anpassungsergebnis zu erhalten.

10. Verfahren nach Anspruch 9, wobei das Bestimmen, ob mindestens ein vorbestimmtes Kriterium von dem überwachten Parameter erfüllt wird, auf dem automatischen Analysieren des Anpassungsergebnisses basiert.

11. Verfahren nach einem der Ansprüche 7 bis 10, das ferner den Schritt des automatischen Kryokonservierens der HCDC-Zubereitungen in der einen oder den mehreren Aufnahmevorrichtungen (7a) umfasst, um kryokonservierte HCDC-Zubereitungen (2) zu erhalten.

12. Verfahren nach einem der Ansprüche 7 bis 11, das ferner, in mindestens einem Iterationsschritt, das Bestimmen der Qualität der erhaltenen HCDC-Zubereitungen und Folgendes umfasst:
- Verändern des vorbestimmten, von dem überwachten Parameter zu erfüllenden Kriteriums, und/oder
- Verändern mindestens eines zweiten Parameters; und
- Wiederholen des Verfahrens nach einem der Ansprüche 8 bis 13, insbesondere, bis eine optimale Qualität erhalten wird.

## Revendications

1. Système (10A ; 10B ; 10C) pour la production automatisée de préparations de culture de semence à haute densité cellulaire, HCDC, à des fins de cryoconservation, comprenant :
- un dispositif de multiplication cellulaire (1) pour faire croître une HCDC (2) dans un milieu (b) à l'intérieur d'un volume de dispositif (V) du dispositif de multiplication cellulaire (1),
- au moins un capteur (3a, 3b) pour la collecte de données par surveillance au moins temporelle d'au moins un paramètre qui est indicatif de l'état physiologique de ladite HCDC ; et
- une unité de commande (5) pour traiter les données collectées afin de déterminer si au moins un critère prédéterminé est ou non rempli par le paramètre surveillé afin de commander, sur la base que l'au moins un critère prédéterminé est ou non rempli par le paramètre surveillé, au moins un échange partiel du milieu (b) par un agent cryoprotecteur, CPA (a) ;
dans lequel le dispositif de multiplication cellulaire (1) comprend
- au moins un drain (1l) pour drainer au moins une portion du milieu (b) depuis le volume de dispositif (V) vers l'extérieur du volume de dispositif (V) ; et
- un élément de membrane (1a) disposé au niveau du drain (1l) de façon que la HCDC (2) soit retenue à l'intérieur du volume de dispositif (V) quand au moins une portion du milieu (b) est drainée depuis le volume de dispositif (V) à travers l'élément de membrane (1a) et à travers le drain (1l) vers l'extérieur du volume de dispositif (V) ; et
dans lequel l'unité de commande (5) est configurée pour initier, sur la base que le critère est ou non rempli, les étapes de
- drainage d'au moins une portion du milieu (b) à l'intérieur du volume de dispositif (V) à travers l'élément de membrane (1a) ; et
- addition du CPA (a) à la HCDC (2) dans le volume de dispositif (V), et
dans lequel le système comprend une unité de transfert (8 ; L3a ; L3b, L3c) commandé par l'unité de commande (5), pour transférer au moins une portion de la HCDC (2) avec le CPA (a) depuis le volume de dispositif (V) dans un ou plusieurs dispositifs récepteurs (7a) afin que soient obtenues des préparations de HCDC pour cryoconservation ; et
dans lequel le système (10A ; 10B ; 10C) comprend en outre :
- une unité de centrifugation (6) pour centrifuger la HCDC (2) avec le milieu (b) ;
- au moins une unité de transfert, commandée par l'unité de commande (5), pour transférer au moins une portion de la HCDC (2) avec le milieu (b) dans au moins un récipient de centrifugation de l'unité de centrifugation (6) ; et
dans lequel l'unité de commande (5) est configurée pour initier, sur la base que le critère est ou non rempli, les étapes de transfert d'au moins une portion de la HCDC (2) avec le milieu (b) depuis le volume de dispositif (V) dans l'au moins un récipient de centrifugation de l'unité de centrifugation (6), centrifugation avec l'unité de centrifugation (6) de la HCDC (2) transférée avec le milieu (b), séparation au moins partiellement du milieu centrifugé (b) d'avec la HCDC (2), et addition d'un agent cryoprotecteur, CPA (a), à la HCDC (2).

2. Système selon la revendication 1, dans lequel l'unité de transfert (8 ; L3a, L3b, L3c) est configurée pour transférer automatiquement au moins une portion de la HCDC (2) avec du CPA (a) depuis le volume de dispositif (V) dans un ou plusieurs dispositifs de réception (7a) pour que soient obtenues des préparations de HCDC pour cryoconservation.

3. Système (10A ; 10B ; 10C) selon l'une quelconque des revendications précédentes, dans lequel le capteur (3a, 3b) forme une unité solidaire avec le dispositif de multiplication cellulaire (1), de préférence dans lequel le capteur (3a, 3b) est soudé dans au moins une portion d'une paroi (1b) du dispositif de multiplication cellulaire (1), et/ou dans lequel le capteur (3a, 3b) s'insère dans le volume de dispositif (V) par l'intermédiaire d'un orifice du dispositif de multiplication cellulaire (1).

4. Système (10A ; 10C) selon l'une quelconque des revendications 1 à 3, dans lequel l'élément de membrane (1a) forme une unité solidaire avec le dispositif de multiplication cellulaire (1).

5. Système (10A ; 10B ; 10C) selon l'une quelconque des revendications précédentes, dans lequel l'unité de commande (5) est configurée pour, sur la base du paramètre surveillé, commander automatiquement au moins un deuxième paramètre, dans lequel le deuxième paramètre est de préférence configuré pour commander et/ou réguler l'état physiologique de la HCDC.

6. Système (10A ; 10B ; 10C) selon l'une quelconque des revendications précédentes, dans lequel au moins l'un parmi le dispositif de multiplication cellulaire (1), le capteur (3a, 3b), l'unité de centrifugation (6), les dispositifs récepteurs comprenant un sac, un tube et/ou un flacon, et au moins une portion de l'unité de transfert (8 ; L3a, L3b, L3c) est un élément à usage unique et/ou un consommable à usage unique.

7. Procédé pour la production automatisée de préparations de culture de semence à haute densité cellulaire, HCDC, à des fins de cryoconservation, comprenant les étapes de
- croissance d'une HCDC (2) dans un milieu (b) à l'intérieur d'un volume de dispositif (V) du dispositif de multiplication cellulaire (1) ;
- collecte de données par surveillance au moins temporelle d'au moins un paramètre, lequel paramètre est indicatif de l'état physiologique de la HCDC (2) ;
- envoi des données collectées à une unité de commande (5) ;
- traitement par l'unité de commande (5) des données collectées comprenant au moins la détermination qu'au moins un critère prédéterminé est ou non rempli par le paramètre surveillé ; et
- commande, sur la base que l'au moins un critère prédéterminé est ou non rempli par le paramètre surveillé, d'au moins un échange partiel du milieu (b) par un agent cryoprotecteur, CPA (a) ;
dans lequel, sur la base que le paramètre surveillé remplit ou non l'au moins un critère :
- l'initiation par l'unité de commande (5) des étapes comprenant
option A)
le drainage via un élément de membrane (1a) pour retenir la HCDC (2) à l'intérieur du volume de dispositif (V) et via un drain (1l) d'au moins une portion du milieu (b) depuis le volume de dispositif (V),
l'addition d'un agent cryoprotecteur CPA (a) à la HCDC (2) dans le volume de dispositif (V), et
le transfert d'au moins une portion de la HCDC (2) dans le CPA (a) depuis le volume de dispositif (V) dans un ou plusieurs dispositifs récepteurs (7a) pour que soient obtenues des préparations de HCDC pour cryoconservation,
dans laquelle l'étape de transfert est effectuée automatiquement ; et/ou
option B)
le transfert d'au moins une portion de la HCDC (2) avec le milieu (b) depuis le volume de dispositif (V) dans au moins un récipient de centrifugation de l'unité de centrifugation (6),
la centrifugation avec l'unité de centrifugation (6) de la HCDC (2) transférée avec le milieu (b),
la séparation au moins partielle du milieu centrifugé (b) d'avec la HCDC (2), et
l'addition de CPA (a) à la HCDC (2) pour que soient obtenues des préparations de HCDC pour cryoconservation,
dans laquelle le transfert d'au moins une portion de la HCDC (2) avec la CPA (a) depuis le récipient de centrifugation dans un ou plusieurs dispositifs de réception (7a) pour que soient obtenues des préparations de HCDC pour cryoconservation, et dans laquelle l'étape de transfert d'au moins une portion de la HCDC (2) avec le milieu (b) depuis le volume de dispositif (V) dans au moins un récipient de centrifugation et/ou de transfert d'au moins une portion de la HCDC (2) avec le CPA (a) depuis le récipient de centrifugation dans le ou les dispositifs récepteurs (7a), sont effectués automatiquement.

8. Procédé selon la revendication 7, comprenant en outre, sur la base du paramètre surveillé, la commande automatisée, par l'unité de commande (5), d'au moins un deuxième paramètre, dans lequel le deuxième paramètre est configuré pour commander et/ou réguler un état physiologique de la HCDC (2).

9. Procédé selon l'une quelconque des revendications 7 et 8, dans lequel le traitement des données collectées comprend l'analyse automatique des données collectées par ajustement d'une fonction et/ou d'un modèle de construction à au moins une portion des données collectées pour que soit obtenu un résultat ajusté.

10. Procédé selon la revendication 9, dans lequel la détermination qu'au moins un critère prédéterminé est ou non rempli par le paramètre surveillé est basée sur l'analyse automatique du résultat ajusté.

11. Procédé selon l'une quelconque des revendications 7 à 10, comprenant en outre l'étape de cryoconservation automatique des préparations de HCDC dans le ou les dispositifs récepteurs (7a) pour que soient obtenues des préparations de HCDC (2) cryoconservées.

12. Procédé selon l'une quelconque des revendications 7 à 11, comprenant en outre, dans au moins une étape d'itération, la détermination de la qualité des préparations de HCDC obtenues et
- la variation du critère prédéterminé devant être rempli par le paramètre surveillé et/ou
- la variation d'au moins un deuxième paramètre ; et
- la répétition du procédé de l'une quelconque des revendications 8 à 13, en particulier jusqu'à ce qu'une qualité optimale soit obtenue.
